(19) Europäisches Patentamt
European Patent Office
Office européen des brevets

(11) EP 1 704 249 B1

(12) **FASCICULE DE BREVET EUROPEEN**

(45) Date de publication et mention de la délivrance du brevet:
**17.11.2010 Bulletin 2010/46**

(21) Numéro de dépôt: **04805634.5**

(22) Date de dépôt: **03.12.2004**

(51) Int Cl.:
***C12Q 1/68*** *(2006.01)*

(86) Numéro de dépôt international:
**PCT/FR2004/003115**

(87) Numéro de publication internationale:
**WO 2005/056828 (23.06.2005 Gazette 2005/25)**

(54) **PROCEDE D'EVALUATION QUANTITATIVE D'UN REARRANGEMENT OU D'UNE RECOMBINAISON GENETIQUE CIBLEE D'UN INDIVIDU ET SES APPLICATIONS**

VERFAHREN ZUR QUANTITATIVEN BEWERTUNG EINER UMORDNUNG ODER EINER GEZIELTEN GENETISCHEN REKOMBINATION BEI EINEM INDIVIDUUM SOWIE VERWENDUNGEN DAVON

METHOD FOR QUANTITATIVE EVALUATION OF A REARRANGEMENT OR A TARGETED GENETIC RECOMBINATION OF AN INDIVIDUAL AND USES THEREOF

(84) Etats contractants désignés:
**AT BE BG CH CY CZ DE DK EE ES FI FR GB GR HU IE IS IT LI LT LU MC NL PL PT RO SE SI SK TR**

(30) Priorité: **05.12.2003 FR 0314289**

(43) Date de publication de la demande:
**27.09.2006 Bulletin 2006/39**

(73) Titulaires:
- **Commissariat à l'Énergie Atomique et aux Énergies Alternatives
75015 Paris (FR)**
- **INSERM (Institut National de la Santé et de la Recherche Medicale)
75013 Paris (FR)**

(72) Inventeurs:
- **MARCHE, Patrice
F-38240 Meylan (FR)**
- **JOUVIN-MARCHE, Evelyne
F-38240 Meylan (FR)**
- **PASQUAL, Nicolas
F-38100 Grenoble (FR)**

(74) Mandataire: **Vialle-Presles, Marie José et al
Cabinet ORES
36, rue de St Pétersbourg
75008 Paris (FR)**

(56) Documents cités:
**WO-A-90/04648 WO-A-2004/033728
FR-A- 2 671 356**

- **PASQUAL NICOLAS ET AL: "Quantitative and qualitative changes in V-J alpha rearrangements during mouse thymocytes differentiation: implication for a limited T cell receptor alpha chain repertoire." THE JOURNAL OF EXPERIMENTAL MEDICINE. 4 NOV 2002, vol. 196, no. 9, 4 novembre 2002 (2002-11-04), pages 1163-1173, XP002322207 ISSN: 0022-1007**
- **PANZARA M A ET AL: "Analysis of the T cell repertoire using the PCR and specific oligonucleotide primers" BIOTECHNIQUES, EATON PUBLISHING, NATICK, US, vol. 12, no. 5, 1992, pages 728-735, XP002028497 ISSN: 0736-6205 cité dans la demande**
- **GLUSMAN G ET AL: "Comparative genomics of the human and mouse T cell receptor loci." IMMUNITY. SEP 2001, vol. 15, no. 3, septembre 2001 (2001-09), pages 337-349, XP002322411 ISSN: 1074-7613 cité dans la demande**
- **VAN DONGEN J J M ET AL: "Design and standardization of PCR primers and protocols for detection of clonal immunoglobulin and T-cell receptor gene recombinations in suspect lymphoproliferations: Report of the BIOMED-2 Concerted Action BMH4-CT98-3936." LEUKEMIA (BASINGSTOKE), vol. 17, no. 12, 1 décembre 2003 (2003-12-01), pages 2257-2317, XP002287366 ISSN: 0887-6924**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **HODGES E ET AL: "Diagnostic role of tests for T cell receptor (TCR) genes." JOURNAL OF CLINICAL PATHOLOGY (LONDON), vol. 56, no. 1, janvier 2003 (2003-01), pages 1-11, XP001196998 ISSN: 0021-9746 cité dans la demande**
- **MANCINI S J ET AL: "TCRA gene rearrangement in immature thymocytes in absence of CD3, pre-TCR, and TCR signaling." JOURNAL OF IMMUNOLOGY (BALTIMORE, MD. : 1950) 15 OCT 2001, vol. 167, no. 8, 15 octobre 2001 (2001-10-15), pages 4485-4493, XP002287367 ISSN: 0022-1767 cité dans la demande**


Remarques:

Le dossier contient des informations techniques présentées postérieurement au dépôt de la demande et ne figurant pas dans le présent fascicule.

SEQUENCE LISTING

<110> COMMISSARIAT A L'ENERGIE ATOMIQUE

INSERM
MARCHE, Patrice
JOUVIN-MARCHE, Evelyne
PASQUAL, Nicolas

<120> PROCEDE D’EVALUATION QUANTITATIVE D’UN REARRANGEMENT OU D’UNE RECOMBINAISON GENETIQUE CIBLEE D’UN INDIVIDU ET SES APPLICATIONS

<130> BLO/clg-F0263/103

<150> FR 0314289
<151> 2003-12-05

<160> 75

<170> PatentIn version 3.1

<210> 1
<211> 26
<212> DNA
<213> Artificial sequence

<220>
<223> amorce PCR

<400> 1
ggtcgttttt cttcattcct tagtcg 26

<210> 2
<211> 24
<212> DNA
<213> Artificial sequence

<220>
<223> amorce PCR

<400> 2
tctcttcatc gctgctcatc ctcc 24

<210> 3
<211> 22
<212> DNA
<213> Artificial sequence

<220>
<223> amorce PCR

<400> 3
tccccttccc attttccact cg 22

<210> 4
<211> 26
<212> DNA
<213> Artificial sequence

<220>
<223> amorce PCR

<400> 4
gcacttacac agacagctcc tccacc 26

<210> 5
<211> 22
<212> DNA
<213> Artificial sequence

<220>
<223> amorce PCR

<400> 5
caggaggaac cagagcccag tc 22

<210> 6
<211> 23
<212> DNA
<213> Artificial sequence

<220>
<223> amorce PCR

<400> 6
tggagtaggg cagggaggac agt 23

<210> 7
<211> 27
<212> DNA
<213> Artificial sequence

<220>
<223> amorce PCR

<400> 7
ggctgggaag tttggtgata tagtgtc 27

<210> 8
<211> 24
<212> DNA
<213> Artificial sequence

<220>
<223> amorce PCR

<400> 8
agcagccaaa tccttcagtc tcaa 24

<210> 9
<211> 28
<212> DNA
<213> Artificial sequence

<220>
<223> amorce PCR

<400> 9
aagacaaaaa ctcccccatt gtgaaata 28

<210> 10
<211> 28
<212> DNA
<213> Artificial sequence

<220>
<223> amorce PCR

<400> 10
gccctcctga aaatgtgtaa agaaatgt 28

<210> 11
<211> 25
<212> DNA
<213> Artificial sequence

<220>
<223> amorce PCR

<400> 11
cttcccccac tcccttcaaa cttac 25

<210> 12
<211> 20
<212> DNA
<213> Artificial sequence

<220>
<223> amorce PCR

<400> 12
agcacttgac ggcagcagca 20

<210> 13
<211> 22
<212> DNA
<213> Artificial sequence

<220>
<223> amorce PCR

<400> 13
tgccccgaga cctgataacc aa 22

<210> 14
<211> 25
<212> DNA
<213> Artificial sequence

<220>
<223> amorce PCR

<400> 14
tcagaacaag ctggaggcaa ctagg 25

<210> 15
<211> 27
<212> DNA

<213> Artificial sequence

<220>
<223> amorce PCR

<400> 15
ggaatagaaa gcgactcact caccagg 27

<210> 16
<211> 25
<212> DNA
<213> Artificial sequence

<220>
<223> amorce PCR

<400> 16
ccacttttag ctgagtgcct gtccc 25

<210> 17
<211> 25
<212> DNA
<213> Artificial sequence

<220>
<223> amorce PCR

<400> 17
ctgtctctgc aatgatgaaa tggcc 25

<210> 18
<211> 21
<212> DNA
<213> Artificial sequence

<220>
<223> amorce PCR

<400> 18
ggaaactctg ggcatgggca g 21

<210> 19
<211> 22
<212> DNA
<213> Artificial sequence

<220>
<223> amorce PCR

<400> 19
actgggcagg agattcggtt at 22

<210> 20
<211> 25
<212> DNA
<213> Artificial sequence

<220>

<223> amorce PCR

<400> 20
cgccccagat taactgatag ttgct 25

<210> 21
<211> 24
<212> DNA
<213> Artificial sequence

<220>
<223> amorce PCR

<400> 21
atactaaggg caggtgaggc tcca 24

<210> 22
<211> 24
<212> DNA
<213> Artificial sequence

<220>
<223> sonde

<400> 22
tcgtttttct tcattcctta gtcg 24

<210> 23
<211> 25
<212> DNA
<213> Artificial sequence

<220>
<223> sonde

<400> 23
atgaaacaag accaaagact cactg 25

<210> 24
<211> 19
<212> DNA
<213> Artificial sequence

<220>
<223> sonde

<400> 24
tgacccagct tgacagcca 19

<210> 25
<211> 22
<212> DNA
<213> Artificial sequence

<220>
<223> sonde

<400> 25

ggcaatcgct gaagacagaa ag 22

<210> 26
<211> 22
<212> DNA
<213> Artificial sequence

<220>
<223> sonde

<400> 26
gagaacaggt gtaagtgccg cc 22

<210> 27
<211> 24
<212> DNA
<213> Artificial sequence

<220>
<223> sonde

<400> 27
ttggattcac ggttaagaga gttc 24

<210> 28
<211> 24
<212> DNA
<213> Artificial sequence

<220>
<223> sonde

<400> 28
tccagtccca aaggttaatt tctc 24

<210> 29
<211> 21
<212> DNA
<213> Artificial sequence

<220>
<223> sonde

<400> 29
ccgaagttga gtgcataccc g 21

<210> 30
<211> 25
<212> DNA
<213> Artificial sequence

<220>
<223> sonde

<400> 30
caaaatcaag gatggctaga aacac 25

<210> 31

<211> 23
<212> DNA
<213> Artificial sequence

<220>
<223> sonde

<400> 31
cttccaaagt atagcctccc cag 23

<210> 32

<211> 22
<212> DNA
<213> Artificial sequence

<220>
<223> sonde

<400> 32
ggtgagtttg tttcctcctc cc 22

<210> 33
<211> 24
<212> DNA
<213> Artificial sequence

<220>
<223> sonde

<400> 33
cccaaaagta agtgctctcc tgcc 24

<210> 34
<211> 22
<212> DNA
<213> Artificial sequence

<220>
<223> sonde

<400> 34
cggggagaag tggaaactct gg 22

<210> 35
<211> 24
<212> DNA
<213> Artificial sequence

<220>
<223> sonde

<400> 35
tcagagttat tccttttcca aatg 24

<210> 36
<211> 25
<212> DNA

<213> Artificial sequence

<220>
<223> sonde

<400> 36
cgccccagat taactgatag ttgct 25

<210> 37
<211> 19
<212> DNA
<213> Artificial sequence

<220>
<223> sonde

<400> 37
ggtccctgct ccaaactgc 19

<210> 38
<211> 50
<212> DNA
<213> Artificial sequence

<220>
<223> séquence amplifiée par PCR

<400> 38
ttgagagat gctgctgtgt actactgcat cctgagagac gggggggggg 50

<210> 39
<211> 65
<212> DNA
<213> Artificial sequence

<220>
<223> séquence amplifiée par PCR

<400> 39

cctttgagg agctccagat gaaagactct gcctcttacc tctgtgctgt gaggaatggg 60

ggggg 65

<210> 40
<211> 41
<212> DNA
<213> Artificial sequence

<220>
<223> séquence amplifiée par PCR

<400> 40
gcgatgtatt tctgtgctta catgagcccg gggggggggg g 41

<210> 41
<211> 328

<212> DNA
<213> Artificial sequence

<220>
<223> séquence amplifiée par PCR

<400> 41

tattctgtat ctgatgatgt ctttgagaac aggtgtaagt gccgccaaaa atgaagtgga 60
gcagagtcct cagaacctga ctgcccagga aggagaattt atcacaatca actgcagtta 120
ctcggtagga ataagtgcct tacactggct gcaacagcat ccaggaggag gcattgtttc 180
cttgtttatg ctgagctcag ggaagaagaa gcatggaaga ttaattgcca caataaacat 240
acaggaaaag cacagctccc tgcacatcac agcctcccat cccagagact ctgccgtcta 300
catctgtgct gtcagagggg gggggggg 328

<210> 42
<211> 44
<212> DNA
<213> Artificial sequence

<220>
<223> séquence amplifiée par PCR

<400> 42
actcagccgt gtactactgt cttctgggag atgggggggg gggg 44

<210> 43
<211> 162
<212> DNA
<213> Artificial sequence

<220>
<223> séquence amplifiée par PCR

<400> 43

acctggagca ggtctccagc ttgctgacgt atattttttc aaatatggac atgaaacaag 60
accaaagact cactgttcta ttgaataaaa aggataacat ctgtctctgc gcattgcaga 120
cacccagact ggggactcag ctatctactt ctgtgcagag ag 162

<210> 44
<211> 215
<212> DNA
<213> Artificial sequence

<220>
<223> séquence amplifiée par PCR

<220>
<221> misc_feature

<222> (22)..(22)
<223> N= A,T,G ou C

<220>
<221> misc_feature
<222> (80)..(82)
<223> N= A,T,G ou C

<220>
<221> misc_feature
<222> (89)..C90)
<223> N= A,T,G ou C

<220>
<221> misc_feature
<222> (146)..(146)
<223> N= A,T,G ou C

<220>
<221> misc_feature
<222> (156)..(156)
<223> N= A,T,G. ou C

<220>
<221> misc_feature
<222> (160)..(160)
<223> N= A,T,G ou C

<220>
<221> misc_feature
<222> (179)..(179)
<223> N= A,T,G ou C

<220>
<221> misc_feature
<222> (186)..(186)
<223> N= A,T,G ou C

<400> 44

```
actactcatc gtctgtttca cngtatctct tctggtatgt gcaatacccc aaccaaggac     60
tccagcttct cctgaagtan nnatcaggnn ccaccctggt taaaggcatc aacggttttg    120
aggctgaatt taacaagagt gaaacntcct tccacntgan gaaaccctca gcccatatna    180
gcgacncggc tgagtacttc tgtgctgtga gtgat                               215
```

<210> 45
<211> 163
<212> DNA
<213> Artificial sequence

<220>
<223> séquence amplifiée par PCR

<400> 45

acctggagca ggtctccagt tgctgacgta tattttttca aatatggaca tgaaacaaga 60

ccaaagactc actgttctat tgaataaaaa ggataaacat ctgtctctgc gcattgcaga 120

cacccagact ggggactcag ctatctactt ctgtgcagag agt 163

<210> 46
<211> 24
<212> DNA
<213> Artificial sequence

<220>
<223> amorce PCR

<400> 46
gcaacatgct ggcggagcac ccac 24

<210> 47
<211> 22
<212> DNA
<213> Artificial sequence

<220>
<223> amorce PCR

<400> 47
atggctttgc agagcactct gg 22

<210> 48
<211> 19
<212> DNA
<213> Artificial sequence

<220>
<223> amorce PCR

<400> 48
gcctctgcac ccatctcga 19

<210> 49
<211> 24
<212> DNA
<213> Artificial sequence

<220>
<223> amorce PCR

<400> 49
gaggatgtgg agcagagtct tttc 24

<210> 50
<211> 24
<212> DNA

<213> Artificial sequence

<220>
<223> amorce PCR

<400> 50
cggccaccct gacctgcaac tata 24

<210> 51
<211> 25
<212> DNA
<213> Artificial sequence

<220>
<223> amorce PCR

<400> 51
gggaccccag cagggagacg ttgcc 25

<210> 52
<211> 24
<212> DNA
<213> Artificial sequence

<220>
<223> amorce PCR

<400> 52
atgctcctgt tgctcatacc agtg 24

<210> 53
<211> 24
<212> DNA
<213> Artificial sequence

<220>
<223> amorce PCR

<400> 53
cctgaaagcc acgaaggctg atga 24

<210> 54
<211> 21
<212> DNA
<213> Artificial sequence

<220>
<223> amorce PCR

<400> 54
gcatctgacg accttcttgg t 21

<210> 55
<211> 24
<212> DNA
<213> Artificial sequence

<220>

<223> amorce PCR

<400> 55
ccatgatgcg gggactggag ttgc 24

<210> 56
<211> 24
<212> DNA
<213> Artificial sequence

<220>
<223> amorce PCR

<400> 56
cattcgttca aatgtgggcg aaaa 24

<210> 57
<211> 24
<212> DNA
<213> Artificial sequence

<220>
<223> amorce PCR

<400> 57
cagaagataa ctcaaaccca acca 24

<210> 58
<211> 21
<212> DNA
<213> Artificial sequence

<220>
<223> amorce PCR

<400> 58
agagtgactc agcccgagaa g 21

<210> 59
<211> 24
<212> DNA
<213> Artificial sequence

<220>
<223> amorce PCR

<400> 59
ccgggcagca gacactgctt ctta 24

<210> 60
<211> 24
<212> DNA
<213> Artificial sequence

<220>
<223> amorce PCR

<400> 60

tcgtcggaac tcttttgatg agca 24

<210> 61
<211> 21
<212> DNA
<213> Artificial sequence

<220>
<223> amorce PCR

<400> 61
gtcttgtggc ttcagcttgg c 21

<210> 62
<211> 24
<212> DNA
<213> Artificial sequence

<220>
<223> amorce PCR

<400> 62
tgcctcgctg gataaatcat cagg 24

<210> 63
<211> 24
<212> DNA
<213> Artificial sequence

<220>
<223> amorce PCR

<400> 63
gggagctctg ctggggctct tgag 24

<210> 64
<211> 20
<212> DNA
<213> Artificial sequence

<220>
<223> amorce PCR

<400> 64
gcagcttccc ttccagcaat 20

<210> 65
<211> 24
<212> DNA
<213> Artificial sequence

<220>
<223> amorce PCR

<400> 65
ggagaggact tcaccacgta ctgc 24

<210> 66

<211> 21
<212> DNA
<213> Artificial sequence

<220>
<223> amorce PCR

<400> 66
ggctggtggc aagagtaact g 21

<210> 67
<211> 24
<212> DNA
<213> Artificial sequence

<220>
<223> amorce PCR

<400> 67
cactgcggcc cagcctggtg atac 24

<210> 68
<211> 24
<212> DNA
<213> Artificial sequence

<220>
<223> amorce PCR

<400> 68
cagcaagtta agcaaaattc acca 24

<210> 69
<211> 22
<212> DNA
<213> Artificial sequence

<220>
<223> amorce PCR

<400> 69
gccgtgatcc tccgagaagg gg 22

<210> 70
<211> 24
<212> DNA
<213> Artificial sequence

<220>
<223> amorce PCR

<400> 70
tgatgatgct acagaaaggt gggg 24

<210> 71
<211> 27
<212> DNA
<213> Artificial sequence

<220>
<223> amorce PCR

<400> 71
ggctgggaag tttggtgata tagtgtc 27

<210> 72
<211> 21
<212> DNA
<213> Artificial sequence

<220>
<223> amorce PCR

<400> 72
atgatgaagt gtccacaggc t 21

<210> 73
<211> 24
<212> DNA
<213> Artificial sequence

<220>
<223> amorce PCR

<400> 73
agcagccaaa tccttcagtc tcaa 24

<210> 74
<211> 28
<212> DNA
<213> Artificial sequence

<220>
<223> amorce PCR

<400> 74
aagacaaaaa ctcccccatt gtgaaata 28

<210> 75
<211> 20
<212> DNA
<213> Artificial sequence

<220>
<223> amorce PCR

<400> 75
cagagttccc cggaccagac 20

## Description

La présente invention est relative à un procédé d'évaluation quantitative d'un réarrangement ou d'une recombinaison génétique ciblée d'un individu et notamment du répertoire immunitaire d'un individu et ses applications, notamment dans le suivi d'un traitement ou dans le diagnostic et/ou le pronostic de certaines pathologies.

Les lymphocytes T et B jouent un rôle prépondérant dans l'adaptation de la réponse immunitaire. Ils sont capables de reconnaître un grand nombre d'antigènes, à l'aide de deux groupes de récepteurs spécifiques : les récepteurs des cellules T (TCR ou T-cell receptors) et les immunoglobulines (Ig). Les TCRs sont stimulés par les antigènes, sous la forme de peptides liés aux molécules du CMH de classe I ou de classe II et présentés aux dits TCRs par les cellules présentatrices des antigènes (1, 2).

Chaque TCR (spécifique d'un antigène) est exprimé à la surface des cellules T et est constitué de deux hétérodimères liés à la membrane et associé au complexe CD3 ; ces hétérodimères sont composés de deux chaînes polypeptidiques $\alpha\beta$ ou $\gamma\delta$ (3), liées entre elles par un pont disulfure ; chaque chaîne comprend un domaine variable (V) et un domaine constant (C). Les domaines $\alpha$ et $\beta$ ou $\gamma$ et $\delta$ des régions variables forment la zone de liaison à l'antigène (4, 5). Les domaines de reconnaissance de l'antigène des TCRs sont générés dans les lymphocytes T au cours de leur différenciation, essentiellement suite à des réactions de recombinaison somatiques ADN-site spécifique, dénommées recombinaisons V(D)J (9, 10). Pendant le développement des cellules T, les chaînes $\alpha$, $\beta$, $\gamma$ et $\delta$ des TCRs sont assemblées suite au réarrangement de leurs gènes idoines, indépendamment des différents loci (TCRAD, TCRB, TCRG). Chez l'Homme, les gènes des TCRs sont localisés sur les chromosomes 7 et 14 : les gènes codant pour la chaîne TCRD sont localisés dans le locus de la chaîne TCRA sur le chromosome 14q11-12, alors que les gènes codant pour les chaînes TCRB et TCRG sont localisés sur le chromosome 7, respectivement aux positions 7q32-35 et 7p15. Ces quatre loci codent pour les domaines constants et variables des quatre chaînes. Les domaines V, dans la chaîne TCRA sont assemblés, à partir de segments de gènes V et J.

Ainsi, l'ensemble des lymphocytes T définissent un répertoire immunitaire, qui constitue le registre des différentes formes des récepteurs et de leur spécificité antigénique. Le répertoire se base sur la grande diversité des structures des récepteurs des antigènes. Pour générer cette diversité, les lymphocytes sont dotés de plusieurs mécanismes dont le principal est la recombinaison V(D)J. Brièvement, les gènes codant pour les récepteurs des antigènes sont discontinus au niveau du génome des cellules de l'organisme de telle sorte que ces gènes sont inactifs. On distingue plusieurs segments de gènes :

a) la région constante (C), qui est commune à tous les récepteurs d'une famille, quelle que soit leur spécificité,
b) la région variable (V), dont le nombre varie suivant la chaîne TCR considérée, de quelques gènes à plusieurs centaines,
c) la région de jonction (J), qui est un gène intermédiaire entre V et C, dont le nombre varie de 1 à plusieurs dizaines, suivant la chaîne TCR considérée et enfin,
d) la région de diversité (D), qui est un petit gène de quelques nucléotides présents seulement dans les chaînes $\beta$ et $\delta$ des TCR et qui s'intercale entre V et J.

Le processus de réarrangement met en oeuvre un complexe enzymatique (la recombinase V(D)J) (14), qui cible spécifiquement les séquences signal de recombinaison (RSS ou *recombination signal sequence*) flanquant les séquences codant les segments de gènes dispersés V et J.

De manière plus précise, les composants principaux de la recombinase sont les produits de deux gènes d'activation de la recombinaison RAG-1 et RAG-2 (15, 16). Les protéines RAG-1 et RAG-2 se lient à des motifs nucléotidiques conservés, dénommés séquences signal de recombinaison (RSS), qui flanquent chaque gène V, D et J (17, 18).

La RSS consensus consiste en une séquence heptamérique, directement adjacente à l'élément codant et en une séquence nonamérique séparée par un bras espaceur comprenant 12 ou 23 nucléotides relativement non conservés. Les sites de jonction sont déterminés par les RSS et la préférence avérée pour la recombinaison entre une RSS avec un bras espaceur de 12 nucléotides avec une RSS avec un bras espaceur de 23 nucléotides assure une recombinaison V(D)J entre deux segments de type différents (V$\alpha$ et J$\alpha$, par exemple) (19, 20).

De manière plus générale, la recombinaison V(D)J comprend un ensemble de réactions enzymatiques incluant des coupures spécifiques, des activités d'exonucléase et de polymérase et des ligatures d'ADN, qui conduit à la formation d'une entité génique fonctionnelle par lymphocyte. Ce gène fonctionnel est constitué de la région C et d'une combinaison d'un V et d'un J (et optionnellement D), qui constitue la marque d'identité moléculaire du lymphocyte et la base moléculaire de la spécificité du récepteur antigénique.

Les lymphocytes disposent ainsi d'un mécanisme sophistiqué pour générer un répertoire TCR diversifié.

En plus du processus de recombinaison (diversité combinatoire), on observe également des délétions ou des insertions au niveau des jonctions V-J ou V-DJ (9, 21). Ces différents mécanismes permettent d'augmenter la diversité du répertoire TCR et donc le nombre d'antigènes reconnus. Ce dernier mécanisme n'est pas codé, mais augmente considérablement le répertoire TCR.

Un autre facteur de diversification est le couplage des chaînes α et β (ou γ et δ) (22) pour former l'hétérodimère TCR.

La plupart des lymphocytes T exprime un TCRαβ clonotypique qui est composé d'un hétérodimère αβ lié à la membrane.

Chaque chaîne contient un domaine constant et un domaine variable, ce dernier étant responsable de la reconnaissance CMH-peptide, qui a lieu de manière toujours similaire par interaction avec les boucles CDR (complementary-determining region) des domaines V.

Plusieurs géométries peuvent être observées, dépendant de la composition des domaines V et du complexe CMH-peptide reconnu. Par exemple, il a été montré que le TCR est positionné dans une orientation diagonale au dessus de la face du complexe peptide-CMH avec les boucles CDR1 et CDR2 de la chaîne TCRα positionnées au dessus de la moitié N-terminale du peptide et les régions correspondantes du TCRβ étant positionnées au niveau de la moitié C-terminale dudit peptide. Un certain nombre des interactions de CDR1 et CDR2 se font avec les résidus du CMH.

Les boucles les plus variables du TCR, à savoir les boucles CDR3 des chaînes TCRα et TCRβ sont positionnées centralement et sont en contact de préférence avec le peptide.

Ainsi, la diversité potentielle des TCRs-αβ générée par recombinaison aléatoire V(D)J a été estimée à environ $10^{15}$ (2, 23) si n'importe quel gène V se réarrange avec n'importe quel gène D et/ou J (2, 23).

La diversité théorique a été surestimée à plusieurs niveaux :

- l'évaluation du répertoire T a été calculée en faisant l'hypothèse que n'importe quel gène V peut se réarranger avec n'importe quel gène D et/ou J.

Cependant, des données récentes obtenues à partir de thymus de souris ont montré que le nombre de recombinaisons V-Jα est considérablement inférieur à celui prédit par le modèle de réarrangement aléatoire et qu'il existe des associations préférentielles entre les segments Vα et Jα en fonction de leur localisation sur le locus aboutissant à une utilisation régulée et coordonnée (24).

- il existe une contrainte dans l'appariement des chaînes α et β pour former des récepteurs hétérodimères, due à des compatibilités de structures entre les différentes sous-unités, ce qui limite également le répertoire.
- dans le thymus, le répertoire généré est soumis aussi bien à une sélection positive qu'à une sélection négative du fait de l'existence d'interactions avec les molécules du CMH, exprimées sur les cellules stromales. Ces sélections réduisent également la taille du répertoire généré, environ d'un facteur 100 et fournissent le profil de diversité des cellules T matures. Ces lymphocytes ($CD4^+$ ou $CD8^+$) migrent vers la périphérie où ils constituent le pool naïf de lymphocytes T circulants.

Le pool de lymphocytes T périphériques est soumis à des mécanismes homéostatiques complexes qui permettent le maintien du nombre et de la fonction des populations de lymphocytes.

Ceci inclut le contrôle du taux de production, la division des cellules matures, le trafic intracellulaire et la mort cellulaire.

L'établissement d'un répertoire T périphérique chez les sujets sains ne s'appuie donc pas uniquement sur les interactions de chaque cellule T avec ses ligands respectifs mais aussi sur une compétition avec les autres sous-populations de lymphocytes.

Ainsi, la taille de la diversité des TCRαβ périphériques est difficile à déterminer.

Des estimations ont récemment été obtenues par extrapolation à partir de mesures moléculaires de la diversité TCR en utilisant l'analyse de la longueur du segment CDR3.

Toutefois, ces analyses concernent principalement la diversité de la chaîne β en raison de la complexité du locus de la chaîne Vα.

Le locus humain TCRAD comprend environ 1 000 kb et consiste en 54 gènes Vα appartenant à 41 familles et 8 pseudogènes, 61 gènes Jα incluant 3 pseudogènes J et un gène unique Cα (25, 26).

En dépit d'un taux élevé d'identité de séquence entre les loci TCR humain et de souris, l'organisation des gènes V et J n'est que partiellement conservée entre les deux espèces.

Alors que le nombre de segments de gènes J pour les chaînes α et β est bien conservé dans les deux espèces, il existe des différences significatives dans le nombre de segments Vα et Vβ.

En particulier, il y a au moins deux fois plus de gènes Vα chez la souris que chez l'homme et 1/3 de plus de gènes Vβ chez l'homme par rapport à la souris.

Pendant l'évolution, le locus TCRAD de souris a été soumis à des processus multiples de duplication impliquant de larges portions du locus, tandis que chez l'homme la duplication ne s'est produite que dans des parties limitées du locus TCRAD (26).

Ces différents processus montrent que les données observées chez la souris ne sont pas directement transposables chez l'homme.

L'analyse du répertoire immunitaire chez l'homme a été proposée, à des fins diagnostiques.

Par exemple, Hodges E et al. (46) résument le rôle diagnostic des tests d'évaluation des gènes des récepteurs des cellules T (TCR). En particulier, les Auteurs de cet article montrent qu'il est maintenant possible d'étudier la pathogenèse d'une maladie au niveau génomique. Le réarrangement génique des récepteurs des cellules T (TCR) est un événement important dans l'ontogénie des cellules T, qui permet aux cellules T de reconnaître les antigènes de manière spécifique.

La moindre dérégulation dans ce processus hautement complexe peut entraîner une maladie.

Cet article fait le point sur les connaissances concernant le mécanisme de réarrangement génique des TCR et décrit les désordres dans lesquels on observe des expansions et des proliférations clonales des cellules T.

Les méthodes actuellement utilisées pour étudier les différentes populations de cellules T, leur rôle diagnostic et les limitations de ces méthodes sont également exposés dans cet article.

Le Tableau I de ce document résume les différents gènes codant pour les chaînes du TCR: $\alpha$(A), $\beta$(B), $\gamma$(G) et $\delta$(D) et montre en particulier la complexité du locus TCRAD. Chez les individus normaux, le répertoire TCR est stable et polyclonal, alors que des populations clonales sont le signe d'une réponse immunitaire spécifique contre une pathologie tumorale ou infectieuse. Ainsi, des populations clonales et oligoclonales sont observées dans des conditions non tumorales, telles que des infections HIV, EBV, des états particuliers, tels que le sujet âgé, l'autoimmunité, l'immunodéficience variable commune (CVID) et l'immunodéficience combinée sévère (SCID), dans laquelle des anomalies des mécanismes intervenant dans la recombinaison des V(D)J ne permettent qu'un nombre limité de réarrangements corrects du gène du TCR. Les Auteurs de cet article considèrent que :

- Pour l'étude du phénotype, la cytométrie de flux, constitue une méthode rapide, relativement peu chère d'analyse du répertoire TCR ; en outre une telle méthode est reproductible et permet une évaluation quantitative de l'expression de différents gènes V dans les différents sous-ensembles de cellules T (cellules CD4+ et cellules CD8+).
- Pour l'étude du génotype, la PCR, qui a largement remplacé l'analyse en Southern blot est utile, pour les études de clonalité, dans la mesure où la PCR offre un certain nombre d'avantages et notamment la rapidité de réalisation et la possible utilisation d'une faible quantité d'ADN.

Plusieurs protocoles de PCR différents peuvent avantageusement être utilisés :

1. L'amplification de tous les segments V, en utilisant des amorces spécifiques de familles complémentaires à tous les gènes TCRV et une amorce située dans la région constante (méthode semi-quantitative uniquement) (Demande de Brevet français n° 2 671 356 ; Panzara M.A. et al., Biotechniques, 1992, 12, 5, 728-735 et Langerak A.W. et al., Blood, 2001, 98, 1, 165-173.
2. une PCR qui inclut une étape de modification de tous les transcrits TCR, de telle sorte qu'ils puissent être amplifiés par un seul oligonucléotide (PCR d'ancrage).
3. une PCR permettant la production de transcrits circulaires par ligation.

Les deux dernières méthodes exigent, pour être mises en oeuvre, une expertise technique de haut niveau (problème d'utilisation en routine), mais permettent une analyse quantitative de la population de cellules T et ont déjà été appliquées aux études de clonalité et à l'évaluation du répertoire TCR dans différentes pathologies.

Il ressort de cet article que les méthodes d'évaluation de la clonalité, en routine doivent de préférence répondre aux caractéristiques suivantes :

- simplicité ;
- robustesse avec de bons rendements de détection et une bonne sensibilité ;
- mise en oeuvre du test sur ADN ;
- augmentation de la détection et de la sensibilité par utilisation d'amorces sélectionnées.

Il ressort également de cet article :

- que l'analyse PCR des gènes TCRG répond à ces conditions (études de clonalité en pathologie clinique), en raison du répertoire limité des gènes TCRG, qui diminue ainsi le nombre d'amorces PCR requises. Toutefois, ce choix a l'inconvénient d'entraîner des amplifications parasites (réarrangements similaires dans les cellules normales) ;
- que l'analyse PCR des gènes TCRD est un outil utile dans l'analyse de certaines tumeurs et notamment des MRD ;
- que le locus TCRB est le locus de choix pour l'établissement de la clonalité des tumeurs exprimant TCR$\alpha\beta$, alors que
- le locus TCRA est trop complexe pour des analyses clonales de l'ADN.

C'est la raison pour laquelle les différentes méthodes d'analyse des répertoires TCR, actuellement proposées, ne préconisent jamais l'analyse du répertoire TCRA :

- Analyse par transfert de Southern (ADN)

Cette technique est lourde à mettre en oeuvre, peu exhaustive et peu quantitative. Elle permet d'évaluer les réarrangements V(D)J, mais elle est peu sensible car elle nécessite plusieurs μg d'ADN, et d'autre part, elle est peu résolutive pour l'identification des gènes V et J. Enfin, elle se prête mal à une automatisation.

- Analyse par Immunoscope® (Brevet US 5,635,354 ; Pannetier et al., Immunol. Today, 1995, 16, 4, 176-181) ; cette méthode est uniquement qualitative.
- Analyse de la diversité des régions de recombinaison dite CDR3 par Immunoscope® (ARNm) (Demande Internationale PCT WO 02/084567).

Actuellement, cette technique est la plus populaire pour l'évaluation de la diversité des récepteurs antigéniques. Elle se base sur l'utilisation des ARNs qui, une fois convertis en ADNc par la réverse transcriptase, sont sujets à des réactions d'amplification par PCR. Les fragments de gènes amplifiés sont courts (quelques dizaines de nucléotides), ils s'étendent entre le gène V et le gène C ou J et incluent la CDR3 qui est la région la plus polymorphe des récepteurs des antigènes. Elle permet d'estimer la diversité générée lors des réarrangements. Elle donne une indication sur la diversité relative d'un répertoire, sans pouvoir quantifier l'ensemble du répertoire. Ses points faibles résident dans :

1) l'utilisation d'ARN qui est un matériel génétique facilement dégradé et nécessitant des conditions opératoires particulières,
2) le fait qu'il peut exister des biais dans l'évaluation des réarrangements dus au fait que tous les gènes V ne sont pas nécessairement transcrits avec la même efficacité,
3) la synthèse d'ADNc peut varier en fonction des transcrits ; cette étape peut donc introduire un biais difficilement contrôlable.

Cette technique nécessite un nombre assez important de réactions d'amplification rendant difficile l'analyse complète de la diversité d'un récepteur. Ainsi, pour la chaîne bêta du TCR humain, il faudrait 25 réactions indépendantes pour les V, puis reprendre chacune d'entre elles avec l'un des 13 J fonctionnels, pour une analyse en électrophorèse sur gel de polyacrylamide dénaturant.

- Analyse de la jonction V-J

La Demande Internationale PCT WO 2004/033728 bénéficiant d'une date de dépôt du 11 octobre 2002, mais publiée le 22 avril 2004, c'est-à-dire postérieurement à la date de dépôt dont bénéficie la présente Demande, décrit une méthode proposant des combinaisons d'amorces de PCR multiplex standards ainsi que des protocoles standardisés de détection de recombinaisons clonales de gènes Ig ainsi que des gènes du récepteur des cellules T (TCR) dans des échantillons où une prolifération de la lignée lymphoïde est suspectée. La méthode décrite met en oeuvre la technique de PCR multiplex dans des conditions de PCR générant un produit dont la taille est comprise entre 300 et 700 pb et de préférence est inférieure à 300 pb (de manière plus préférée entre 100-300 pb) ; plus précisément, la PCR mise en oeuvre comporte une étape d'élongation de 1,30 min permettant une amplification de 1 à 2 Kb au maximum et rendant impossible l'amplification des hautes tailles nécessaires au multiplexage « naturel » ; en outre, la position des oligonucléotides utilisés cible des régions conservées entre les gènes V ; les différents gènes qui ont été testés dans cette méthode sont les suivants : TCRB, TCRG, TCRD. Ceux-ci ne comprennent pas le gène TCRA qui a été volontairement écarté, en raison de sa complexité.

Cette méthode permet la caractérisation des amplifications monoclonales ; par contre, elle ne permet pas d'avoir une résolution de l'ensemble du répertoire. Cette approche donne donc un résultat de type on/off, peu résolutif, caractérisant une amplification monoclonale, mais en aucun cas ne permet de visualiser l'ensemble du répertoire sous la forme d'une cartographie.

Cette technique ne permet donc pas de connaître la fréquence relative des réarrangements du répertoire du fait de l'étude de la jonction V-S uniquement et n'apporte pas d'aspect différenciateur au niveau d'un réarrangement donné. En effet, le fait d'avoir multiplexé tous les oligonucléotides dans un même tube, sans éviter la superposition des différents produits de PCR, ne permet que d'obtenir un produit d'une taille de 200 à 700 pb pour l'ensemble des réarrangements attendus et empêche d'assigner une bande à un réarrangement particulier. En aucun cas cette technique utilise le principe de multiplexage « naturel » des locis du TCR.

- Cytométrie de flux (voir Hodges E. et al. précité, 46)

Cette technique utilise des anticorps marqués à la fluorescéine, spécifiques des régions V des récepteurs. Elle présente l'intérêt de mesurer l'expression du récepteur antigénique à la surface du lymphocyte. Cependant, elle est très limitée :

. par le manque d'anticorps qui ne permet pas une analyse complète du répertoire ;
. par sa faible sensibilité qui nécessite des échantillons cellulaires de taille importante (plusieurs dizaines de millions de lymphocytes) ;
. sa faible résolution puisqu'elle ne permet que d'identifier le V sans donner d'information sur les combinaisons avec les J ;
. la nécessité de travailler avec des cellules.

En conséquence, la présente invention s'est donnée pour but de pourvoir à un procédé d'évaluation de réarrangements ou de recombinaisons de gènes et notamment du répertoire TCR, qui réponde mieux aux besoins de la pratique que les procédés de l'art antérieur précités.
Pour obtenir une méthode quantitative, simple à mettre en oeuvre, robuste, fiable et quantitative, la présente invention préconise d'utiliser dans des conditions définies, un nombre restreint de réactions enzymatiques de polymérisation en chaîne de l'ADN, dites de « longue PCR » (LPCR) permettant de détecter plusieurs réarrangements de gènes et notamment plusieurs réarrangements V(D)J des gènes codant les TCRAD directement au niveau de l'ADN génomique avec une seule réaction enzymatique (PCR multiplex).
L'amplification d'ADNg pour détecter en une seule étape les réarrangements des gènes V(D)J codant pour les récepteurs spécifiques des antigènes est décrite. L'objet décrit peut être appliqué à d'autres évènements de réarrangements de gènes ou de recombinaisons génétiques ayant des sites définis.
En conséquence, un procédé d'évaluation de toute recombinaison génétique ciblée, est également décrit.
Un procédé d'évaluation quantitative d'un réarrangement ou d'une recombinaison génétique ciblée chez un individu est donc décrit, lequel procédé est **caractérisé en ce qu**'il comprend au moins :

(a) l'extraction de l'ADN génomique humain, à partir d'un échantillon biologique,
(b) l'amplification d'un segment dudit ADN génomique, de taille comprise entre quelques centaines de paires de bases et plusieurs dizaines de kb, par PCR multiplex, en présence :

* d'un ou plusieurs couples d'amorces, sélectionnées de manière à répondre aux caractéristiques suivantes :

- au moins l'une desdites amorces de l'un desdits couples d'amorces, s'hybride en amont et/ou à l'extrémité 5' d'un gène Vx à amplifier, susceptible d'intervenir dans ledit réarrangement génétique ;
- au moins l'autre desdites amorces de l'un desdits couples d'amorces s'hybride en aval et/ou à l'extrémité 3' d'un gène Jy à amplifier, susceptible d'intervenir dans ledit réarrangement génétique ;

* et d'une ADN polymérase ou d'un mélange d'ADN polymérases permettant l'amplification de segments d'ADN génomique de taille comprise entre quelques centaines de paires de bases et plusieurs dizaines de kb, de préférence de taille supérieure à 10 kb et ayant une activité de correction permettant d'améliorer sensiblement l'élongation ;
ladite amplification comprenant outre l'étape de dénaturation initiale, des cycles de dénaturation, d'hybridation et d'élongation et, dans lesquels les étapes d'élongation sont effectuées au moins pendant 10 minutes à 68˚C-72˚C ;

(c) la séparation des fragments d'ADNg amplifiés et
(d) la détection des segments réarrangés ou recombinés.

On entend par individu, de préférence les mammifères pour lesquels il existe un intérêt à déterminer des profils d'expression TCRA (homme, autres primates, animaux domestiques, animaux utilisés dans la chaîne alimentaire, tels que boeuf, mouton, porc...).
Dans le cas où le réarrangement ou la recombinaison génétique ciblée concerne les récepteurs TCRAD, ledit procédé comprend :

(a) l'extraction de l'ADN génomique humain, à partir d'un échantillon biologique,
(b) l'amplification d'un segment dudit ADN génomique, de taille comprise entre quelques centaines de paires de bases et plusieurs dizaines de kb, par PCR longue multiplex, en présence :

* d'un ou plusieurs couples d'amorces, sélectionnées de manière à répondre aux caractéristiques suivantes :

- au moins l'une desdites amorces de l'un desdits couples d'amorces, dénommée amorce V, s'hybride de manière spécifique avec une région située en amont de la séquence RSS d'un gène Vx à amplifier, correspondant à un segment V du domaine variable de la chaîne $\alpha$ d'un récepteur des cellules T (TCRAD) ;
- au moins l'une desdites amorces de l'un desdits couples d'amorces, dénommée amorce J s'hybride de manière spécifique avec une région située en aval de la séquence RSS d'un gène Jy à amplifier, avec l'extrémité 3' dudit gène Jy à amplifier ou dans ledit gène Jy à amplifier, correspondant à un segment J de la chaîne $\alpha$ d'un récepteur des cellules T ;

* et d'une ADN polymérase ou d'un mélange d'ADN polymérases permettant l'amplification de segments d'ADN génomique de taille comprise entre quelques centaines de paires de bases et plusieurs dizaines de kb, de préférence de taille supérieure à 10 kb et ayant une activité de correction permettant d'améliorer sensiblement l'élongation ;
ladite amplification comprenant outre l'étape de dénaturation initiale, des cycles de dénaturation, d'hybridation et d'élongation et, dans lesquels les étapes d'élongation sont effectuées au moins pendant 10 minutes à 68°C-72°C ;

(c) la séparation des fragments d'ADNg amplifiés et
(d) la détection des segments V(D)J recombinés.

Ce procédé présente un certain nombre d'avantages :

- il permet une analyse des combinaisons V(D)J au niveau du génome, ce que ne permet pas la technique de l'Immunoscope®.
- il permet d'obtenir des fragments de grande taille, chaque fragment $V_x$-$J_y$, $V_x$-$J_{y+1}$, $V_x$-$J_{y+2}$ etc... pouvant être clairement distingué des autres, ce qui implique une bonne résolution.
- il permet ainsi l'analyse de l'appariement V/J en tant que tel.
- il permet une analyse moléculaire au niveau de l'ADN génomique.
  En mesurant la quantité d'une combinaison donnée de gènes V(D)J, on peut avoir une évaluation du nombre de lymphocytes possédant ce réarrangement, puisqu'il y a proportionnalité entre le nombre de gènes réarrangés et le nombre de lymphocytes : un lymphocyte ne peut avoir que deux réarrangements pour un type de récepteur, soit un réarrangement par chromosome. Ce type d'information n'est pas accessible par l'analyse de la diversité des régions de recombinaison dite CDR3, ni par l'analyse de la jonction V/J.
- il autorise une évaluation exhaustive de la diversité de récepteurs antigéniques, ce que ne permet ni la cytométrie de flux, ni la technique de Southern.
- il permet de diminuer sensiblement le nombre de réactions de PCR pour une analyse complète du répertoire. Ceci est possible grâce à l'analyse simultanée de plusieurs gènes V et J dans une même réaction d'amplification et une même piste d'électrophorèse. Par exemple, pour la chaîne TCR alpha chez l'homme, il faut 3355 réactions correspondant aux différentes combinaisons VJ pour détecter et quantifier les réarrangements ; le procédé selon l'invention permet de réaliser l'analyse avec 10 fois moins de réactions. En particulier, la combinaison des étapes du procédé selon l'invention et plus particulièrement la combinaison de l'étape (c) de séparation avec l'étape (b) d'amplification, coopère pour améliorer, de manière significative, les performances du procédé selon l'invention, notamment en sélectionnant le système de séparation le plus adapté.
- il permet de quantifier les réarrangements par mesure de l'intensité relative des bandes d'électrophorèse issues d'une même réaction et qui sont analysées

dans une même piste d'électrophorèse. Grâce à une standardisation tenant compte des différences de taille, il est possible en particulier de normaliser les valeurs et d'obtenir la quantification de la proportion de chaque réarrangement par rapport à l'ensemble (très bonne résolution). Aucune technique ne permet actuellement d'obtenir ces résultats.

- il permet l'identification des gènes V et J du récepteur, l'identité du gène V est donnée par l'amorce utilisée lors de l'amplification, l'identité du gène J est obtenue par mesure de la taille du fragment amplifié avec l'amorce J utilisée et par référence avec la séquence nucléotidique publiée. Cette opération est facilitée par l'utilisation de logiciels d'alignement de séquences et de comparaison de motifs tels que Blast, (www.ensembl.org), IMGT/GeneInfo (http:

//imgt.cines.fr/GeneInfo) etc.. Cette information peut être obtenue par l'analyse par l'Immunoscope®, mais l'identification du segment J se fait au cas par cas, alors qu'avec le procédé selon l'invention, plusieurs segments J peuvent être identifiés lors d'une même réaction.

- il permet de révéler des combinaisons V(D)J particulièrement abondantes dans un mélange. Ceci peut se révéler utile pour détecter une réponse spécifique immuno-dominante, c'est-à-dire due à un (réponse monoclonale) voire à quelques clones (réponse oligoclonale) de lymphocytes. En outre, ceci peut servir à la détection de proliférations de lymphocytes telles que celles observées dans les cancers de lymphocytes (lymphomes, myélomes, leucémies). Les leucémies provenant de lymphomes B peuvent être détectées par une analyse directe des Ig circulant au niveau du sérum de sang. Mais la détection des leucémies ne produisant pas d'Ig dans le sérum et celle des leucémies des lymphomes T restent difficiles et peu quantitatives. Le procédé selon l'invention permet d'identifier les combinaisons V(D)J anormalement abondantes et ainsi permet la détection de proliférations dues à des leucémies. Par l'analyse au niveau du génome, le procédé selon l'invention autorise une détection plus facile et sensible du nombre de cellules cancéreuses, dérivées de lymphocytes B et/ou T, et par conséquent, permet d'apporter un diagnostic plus précoce et plus exhaustif par rapport aux techniques déjà existantes telles que la technique de Southern ou l'Immunoscope® de la chaîne TCR beta.

L'étape (a) d'extraction est réalisée par des techniques connues qui respectent les précautions définies pour la purification de l'ADNg utilisées pour la technique de transfert de type Southern (voir Sambrook J., Fritsch EF., Maniatis T., 1989, Molecular cloning. A laboratory manual. Second Edition. Cold Spring Harbor, New York, EUA). Elle peut être avantageusement suivie d'une étape de purification de l'ADNg par les techniques classiques de biologie moléculaire.

Selon un mode de mise en oeuvre avantageux de l'étape (b) d'amplification, la sélection des amorces est effectuée :

- par analyse systématique de l'ensemble du locus concerné et notamment du locus TCRAD humain à l'aide d'un logiciel convenable d'alignement de séquences et de comparaison de motifs, tels que définis ci-dessus et notamment à l'aide d'un logiciel de recherche de séquence homologue (NTI Vector suite 8.0 » de la société Informax Inc., par exemple),
- sélection des amorces dont l'extrémité 3'OH est complémentaire uniquement de la région d'intérêt, telle que définie ci-dessus,
- élimination des amorces formant des auto-dimères ou des épingles à cheveux stables, notamment par une analyse à l'aide d'un logiciel convenable d'alignement de séquences et de comparaison de motifs, et notamment à l'aide d'un logiciel de recherche de séquence homologue (logiciel « vector NTI suite 8.0 » de la société Informax Inc., par exemple), et
- élimination des couples d'amorces formant des hybrides entre eux.

Ainsi, l'ensemble de ces différentes étapes de sélection diminuent les biais de la PCR comme la compétition entre les amorces ou avec d'autres séquences cibles que celles sélectionnées et par conséquent améliore le rendement et la spécificité de la PCR nécessaire pour la réalisation des analyses des réarrangements en PCR longue multiplex.

Les amorces et notamment les amorces V et J des couples d'amorces V/J sont sélectionnées dans le groupe constitué par les amorces illustrées au Tableau I ci-après:

| Espèce Locus | HUMAIN | | | |
|---|---|---|---|---|
| **TRAV** | TCRAD | S/A | bp | tm |
| hTRAV1 | GGTCGTTTTTCTTCATTCCTTAGTCG (SEQ ID NO:1) | S | 27 | **58** |
| hTRAV2 | TCTCTTCATCGCTGCTCATCCTCC (SEQ ID NO:2) | S | 24 | **61** |
| hTRAV3 | TCCCCTTCCCATTTTCCACTCG (SEQ ID NO:3) | S | 22 | **60** |
| hTRAV5 | GCACTTACACAGACAGCTCCTCCACC (SEQ ID NO:4) | S | 26 | **61** |
| hTRAV8 | CAGGAGGAACCAGAGCCCAGTC (SEQ ID NO:5) | S | 22 | **59** |
| hTRAV26-2 | TGGAGTAGGGCAGGGAGGACAGT (SEQ ID NO:6) | S | 23 | **60** |
| hTRAV35 | Ggctgggaagtttggtgatatagtgtc (SEQ ID NO:7) | S | 27 | **59** |
| hTRAV38.2 | AGCAGCCAAATCCTTCAGTCTCAA (SEQ ID NO:8) | S | 24 | **58** |
| hTRAV40 | Aagacaaaaactcccccattgtgaaata (SEQ ID NO:9) | S | 26 | **60** |
| hTRAV41 | GCCCTCCTGAAAATGTGTAAAGAAATGT (SEQ ID NO:10) | S | 28 | **60** |

(suite)

| | | | | |
|---|---|---|---|---|
| **TRAJ_do** | | | | |
| hAJ53do | CTTCCCCCACTCCCTTCAAACTTAC (SEQ ID NO:11) | A | 25 | **60** |
| hAJ48do | AGCACTTGACGGCAGCAGCA (SEQ ID NO:12) | A | 20 | **60** |
| hAJ41do | TGCCCCGAGACCTGATAACCAA (SEQ ID NO:13) | A | 22 | **61** |
| hAJ29do | TCAGAACAAGCTGGAGGCAACTAGG (SEQ ID NO:14) | A | 25 | **61** |
| hAJ18do | GGAATAGAAAGCGACTCACTCACCAGG (SEQ ID NO:15) | A | 27 | **60** |
| hAJ10do | CCACTTTTAGCTGAGTGCCTGTCCC (SEQ ID NO:16) | A | 25 | **60** |
| hAJ5do | CTGTCTCTGCAATGATGAAATGGCC (SEQ ID NO:17) | A | 25 | **59** |
| hAJ1do | GGAAACTCTGGGCATGGGCAG (SEQ ID NO:18) | A | 21 | **59** |
| hAJ56do | ACTGGGCAGGAGATTCGGTTAT (SEQ ID NO:19) | | | |
| hAJ33do | CGCCCCAGATTAACTGATAGTTGCT (SEQ ID NO:20) | | | |
| hAJ24do | ATACTAAGGGCAGGTGAGGCTCCA (SEQ ID NO:21) | | | |

Ainsi, les amorces sélectionnées à l'étape (b) permettent d'obtenir des résultats de qualité : la spécificité des produits de PCR est plus particulièrement établie pour 10 amorces V (SEQ ID NO :1-10), par séquençage des produits d'amplification qui montre la présence d'une séquence unique correspondant au gène V ciblé (voir Tableau III) et la robustesse du choix des amorces.

La spécificité des amorces peut être suivie en utilisant des sondes oligonucléotidiques, telles que celles définies dans le Tableau II ci-après, marquées qui reconnaissent des séquences internes au produit amplifié ; par hybridation il est ainsi possible de vérifier si la taille observée du fragment amplifié correspond à la taille attendue déduite de la séquence du locus TCR alpha humain, en considérant la distance séparant les amorces V et J et leur réarrangement. Ces sondes sont également avantageusement utilisées à l'étape (d) du procédé décrit.

Tableau II

| | | S/A | bp | tm |
|---|---|---|---|---|
| | **Sondes** | | | |
| **TRAV** | | | | |
| hTRAV1p | TCGTTTTTCTTCATTCCTTAGTCG (SEQ ID NO:22) | | | |
| hTRAV5p | ATGAAACAAGACCAAAGACTCACTG (SEQ ID NO:23) | | | |
| hTRAV8p | TGACCCAGCTTGACAGCCA (SEQ ID NO:24) | | | |
| hTRAV26-2p | GGCAATCGCTGAAGACAGAAAG (SEQ ID NO:25) | | | |
| hTRAV4Ip | GAGAACAGGTGTAAGTGCCGCC (SEQ ID NO:26) | | | |
| **TRAJ** | | | | |
| hAJ53p | TTGGATTCACGGTTAAGAGAGTTC (SEQ ID NO:27) | A | 24 | **59** |
| hAJ48p | TCCAGTCCCAAAGGTTAATTTCTC (SEQ ID NO:28) | A | 24 | **59** |
| hAJ41p | CCGAAGTTGAGTGCATACCCG (SEQ ID NO:29) | A | 21 | **55** |
| hAJ29p | CAAAATCAAGGATGGCTAGAAACAC (SEQ ID NO:30) | A | 25 | **55** |
| hAJ18p | CTTCCAAAGTATAGCCTCCCCAG (SEQ ID NO:31) | A | 23 | **55** |
| hAJ10p | GGTGAGTTTGTTTCCTCCTCCC (SEQ ID NO:32) | A | 22 | **57** |
| hAJ5p | CCCAAAAGTAAGTGCTCTCCTGCC (SEQ ID NO:33) | A | 24 | **55** |
| hAJ1p | CGGGGAGAAGTGGAAACTCTGG (SEQ ID NO:34) | A | 22 | **53** |
| hAJ56p | TCAGAGTTATTCCTTTTCCAAATG (SEQ ID NO:35) | | | |
| hAJ33p | CGCCCCAGATTAACTGATAGTTGCT (SEQ ID NO:36) | | | |
| hAJ24p | GGTCCCTGCTCCAAACTGC (SEQ ID NO:37) | | | |

Les sondes et amorces décrites peuvent être marquées directement ou indirectement par un composé radioactif ou non radioactif par des méthodes bien connues de l'Homme du Métier, afin d'obtenir un signal détectable et/ou quantifiable ; le marquage des amorces ou des sondes selon l'Invention est réalisé par des éléments radioactifs ou par des molécules non radioactives. Parmi les isotopes radioactifs utilisés, on peut citer le $^{32}P$, le $^{33}P$, le $^{35}S$ ou le $^{3}H$. Les entités non radioactives sont sélectionnées parmi les ligands tels que la biotine, l'avidine, la streptavidine, la digoxygénine, les haptènes, les colorants, les agents luminescents tels que les agents radioluminescents, chémoluminescents, bioluminescents, fluorescents, phosphorescents.

Pour éviter l'amplification de séquences voisines hors du locus TCRAD, et donc pour diminuer la non spécificité, l'analyse systématique sur l'ensemble du locus TCRAD humain effectuée pour sélectionner les amorces, à l'étape (b) est, de préférence, effectuée par recherche sur tout le génome humain en utilisant la base de données **www.ensembl.org**, qui permet de visualiser l'ensemble des fixations sur le génome humain avec un code couleur de 3 intensités : Rouge = 100 % Vert =85 Bleu = 70.

Seuls les oligonucléotides candidats reconnaissant le locus TCRAD en rouge et ayant un nombre limité de fixations sur le reste du génome (en vert ou bleu) sont retenus.

Les produits d'amplification par PCR utilisant les amorces précisées dans le Tableau III ci-après fournissent les séquences amplifiées précisées dans la colonne de droite.

Tableau III

| oligo up | oligo do | Séquences V amplifiées : SEQ ID NO:38-45 |
|---|---|---|
| SEQ ID NO:6 | SEQ ID NO:11 | CTTGAGAGATGCTGCTGTGTACTACTGCATCCTGAGAGACGGGGGGGGG |
| SEQ ID NO:1 | SEQ ID NO:16 | CCTTTTGAGGAGCTCCAGATGAAAGACTCTGCCTCTTACCTCTGTGCTGTGAGGAATGGGGGGG |
| SEQ ID NO:8 | SEQ ID NO:11 | GCGATGTATTTCTGTGCTTACATGAGCCCGGGGGGGGGGGG |
| SEQ ID NO:10 | SEQ ID NO:11 | TATTCTGTATCTGATGATGTCTTTGAGAACAGGTGTAAGTGCCGCCAAAA<br>ATGAAGTGGAGCAGAGTCCTCAGAACCTGACTGCCCAGGAAGGAGAATTT<br>ATCACAATCAACTGCAGTTACTCGGTAGGAATAAGTGCCTTACACTGGCT<br>GCAACAGCATCCAGGAGGAGGCATTGTTTCCTTGTTTATGCTGAGCTCAG<br>GGAAGAAGAAGCATGGAAGATTAATTGCCACAATAAACATACAGGAAAAG<br>CACAGCTCCCTGCACATCACAGCCTCCCATCCCAGAGACTCTGCCGTCTA<br>CATCTGTGCTGTCAGAGGGGGGGGGGGG |
| SEQ ID NO:9 | SEQ ID NO:11 | |
| SEQ ID NO:4 | SEQ ID NO:16 | ACTCAGCCGTGTACTACTGTCTTCTGGGAGATGGGGGGGGGGG<br>ACCTGGAGCAGGTCTCCAGCTTGCTGACGTATATTTTTTCAAATATGGAC<br>ATGAAACAAGACCAAAGACTCACTGTTCTATTGAATAAAAAGGATAACAT<br>CTGTCTCTGCGCATTGCAGACACCCAGACTGGGGACTCAGCTATCTACTT<br>CTGTGCAGAGAG |
| SEQ ID NO:5 | SEQ ID NO:11 | ACTACTCATCGTCTGTTTCACNGTATCTCTTCTGGTATGTGCAATACCCC<br>AACCAAGGACTCCAGCTTCTCCTGAAGTANNNATCAGGNNCCACCCTGGT<br>TAAAGGCATCAACGGTTTTGAGGCTGAATTTAACAAGAGTGAAACNTCCT<br>TCCACNTGANGAAACCCTCAGCCCATATNAGCGACNCGGCTGAGTACTTC<br>TGTGCTGTGAGTGAT |
| SEQ ID NO:4 | SEQ ID NO:11 | ACCTGGAGCAGGTCTCCAGTTGCTGACGTATATTTTTTCAAATATGGACA<br>TGAAACAAGACCAAAGACTCACTGTTCTATTGAATAAAAAGGATAAACAT<br>CTGTCTCTGCGCATTGCAGACACCCAGACTGGGGACTCAGCTATCTACTT<br>CTGTGCAGAGAGT |

Pour chaque gène Vx, il est préférable de mettre en oeuvre, les couples d'amorces comprenant l'amorce V correspondant audit gène Vx en association avec chacune des amorces J définies ci-dessus (SEQ ID NO:11 à 22). L'étape (b) d'amplification est alors réalisée en parallèle avec les différents couples d'amorces.

Selon un autre mode de mise en oeuvre du procédé décrit, l'étape (b) peut avantageusement mettre en oeuvre des amorces supplémentaires pour amplifier en outre au moins l'un des segments suivants : segments D, segments V et J des chaînes TCR β, γ, δ et éventuellement segments des chaînes des immunoglobulines.

Ainsi, notamment dans le cas des chaînes TCR ou Ig possédant un segment D de diversité, une réaction de PCR entre le gène V et D est effectuée en plus de la réaction V-J commune à tous les réarrangements. Cette étape permet de connaître à la fois le répertoire des segments D et J utilisés en conjonction avec les gènes V. Il en est de même pour les gènes D, dans le cas de l'analyse des chaînes qui ont des D en plus des V et J comme les chaînes TCR bêta et TCR delta et la chaîne lourde des Immunoglobulines.

Les ADN polymérases, mises en oeuvre à l'étape (b) d'amplification sont de préférence un mélange d'ADN polymérases permettant d'amplifier de très longues molécules d'ADN, du type de celui décrit dans le Brevet américain US 6,410,277 ; un tel mélange d'enzymes présente une activité de correction permettant d'améliorer sensiblement l'élongation. Ainsi, une amplification de plusieurs kb d'ADN permet de détecter les recombinaisons successives ayant eu lieu avec tous les gènes potentiels situés entre les deux amorces utilisées.

Selon un autre mode réalisation avantageux de l'étape (b), la réaction de PCR longue multiplex (LPCR) est effectuée après purification de l'ADN par les techniques classiques de biologie moléculaire ou directement sur un lysat cellulaire.

Il est cependant préférable de travailler avec l'ADNg le plus pur possible (exempt de protéines telles que les nucléosomes) pour favoriser une amplification optimum des produits de grande taille (> à 10 kb) ; pour cela, il est important de ne pas casser l'ADN génomique.

Une fois la purification de l'ADN effectuée (soit par une méthode d'extraction au phénol/chloroforme V/V bien connue en biologie moléculaire, soit avec des trousses commercialisées permettant l'extraction et la purification de l'ADN), celui-ci est directement utilisé pour la LPCR multiplex.

Un tube de réaction (volume réactionnel de 20 à 50 μl) est composé d'eau ultra pure, de l'enzyme Taq ou du mélange d'enzymes de polymérisation de l'ADN, tel que celui décrit dans le Brevet US 6,410,277 (mélange dénommé LATaq), de $MgCl_2$ (co-facteur de l'enzyme Taq), des dNTP, de l'amorce oligonucléotidique spécifique du sens de transcription des gènes V à étudier (l'une des amorces de SEQ ID NO:1-10), l'amorce oligonucléotidique spécifique du sens inverse de transcription du segment de gène D ou J (l'une des amorces de SEQ ID NO :11-21) à étudier, de l'ADN à analyser, aussi appelé matrice, à partir de laquelle l'amplification a lieu. Un deuxième oligonucléotide correspondant à une séquence interne du gène V, située en aval de la première amorce V et en amont du RSS du gène V et/ou un deuxième nucléotide J correspondant à une séquence interne du gène J, située en amont de la première amorce J et en aval du RSS du gène J, peuvent être ajoutés à la moitié de la réaction de LPCR pour diminuer le bruit de fond de PCR ; ces oligonucléotides peuvent être marqués ou pas avec un fluorochrome pour permettre la détection directe des produits amplifiés. Le programme du thermocycleur permettant d'effectuer la réaction d'amplification peut être variable en fonction des conditions souhaitées.

On effectue une PCR (étape (b) d'amplification) par couple d'amorces V/J, tel que défini ci-dessus.

Selon un autre mode de mise en oeuvre avantageux de l'étape (b) d'amplification, les étapes d'élongation, sont avantageusement incrémentées de 15-20 secondes par cycle supplémentaire d'élongation.

Toutefois, pour permettre l'amplification de fragments longs d'ADN, il est nécessaire (étape (b)) :

. d'exposer l'ADN génomique à amplifier à des températures supérieures à 90°C (dénaturation) pendant des temps très courts (de l'ordre de 10 à 30 secondes), et ce pour éviter l'acidification du milieu et la dégradation de l'ADN,

. de tenir compte de la « fatigue » de l'ADN polymérase mise en oeuvre, en incrémentant de 15-20 secondes par cycle supplémentaire l'étape d'élongation, notamment à partir du milieu de la PCR.

Un exemple, non limitatif, permettant l'amplification de produits de l'ordre de 10 kb à 20 kb, est décrit ci-après :

- Dénaturation initiale 1-2 minutes à 94°C
- 15 cycles successifs comprenant : une étape de dénaturation : 5 à 30 secondes à 90-94°C, une étape d'hybridation des amorces : 15-30 secondes à 58-62°C et une étape d'élongation : 14 à 20 minutes à 68-72°C et
- 15 cycles successifs comprenant : une étape de dénaturation : 5 à 30 secondes à 90-94°C, une étape d'hybridation des amorces : 15-30 secondes à 58-62°C et une étape d'élongation : 14 à 20 minutes à 68-72°C + 15-20 secondes additionnelles à chaque cycle supplémentaire et
- 1 cycle final d'élongation de 10 minutes à 72°C.

Selon un autre mode de mise en oeuvre avantageux dudit procédé, l'étape (c) de séparation des fragments d'ADN amplifiés est effectuée par migration électrophorétique sur gel d'agarose ou de polyacrylamide, de préférence en champ pulsé.

Les conditions de séparation des produits de PCR peuvent varier en fonction des conditions souhaitées. Voici une condition permettant de séparer des produits de l'ordre de 15 kb par migration sur gel d'électrophorèse d'agarose en TBE 1X (cette étape est bien connue dans le domaine de l'art) :

> Dépôt de 5-20 μl de produit de LPCR sur un gel d'agarose 1,2 % (P/V), migration 100-150 volts pendant 8 h avec un générateur. Il est possible d'utiliser une migration en champs pulsés pour améliorer la séparation des produits de grande taille.

Selon un autre mode de mise en ouvre avantageux dudit procédé, l'étape (c) de séparation des fragments d'ADN amplifiés est effectuée par séparation en microcapillaire sur bioanalyseur. Plus précisément, pour séparer des fragments de LPCR de taille supérieure à 10 kb, on peut utiliser une migration en microcapillaire Bio-analyseur AGILENT Technologies. On peut envisager dans un tel cas, de travailler avec des amorces marquées avec un fluorochrome (par exemple le Cy-5) pour augmenter le signal de détection des fragments amplifiés, à l'étape (d) de détection. La puce AGILENT est préparée et utilisée d'après les indications du constructeur, dépôt de 1 μl du produit de PCR, quantification conformément au guide d'utilisation de l'appareil.

De manière surprenante, la combinaison des éléments suivants de l'étape (b) :

- le choix des séquences des amorces permettant d'étudier les réarrangements des récepteurs antigéniques chez l'homme ;
- le programme du thermocycleur lors des réactions d'amplification ;
- le choix des enzymes utilisées pour améliorer l'amplification des fragments de grande taille ;

  et le protocole de séparation des fragments amplifiés de l'étape (c), permettent effectivement une évaluation quantitative des recombinaisons à détecter.

Dans ce contexte il est possible de détecter entre 1 et 11 réarrangements par réaction PCR et pour visualiser 100 % des réarrangements effectués dans la région J, un minimum de 11 PCR, pour un gène V donné est suffisant.

Selon un autre mode de mise en oeuvre dudit procédé, l'étape (d) de détection peut être avantageusement réalisée à l'aide de l'une des méthodes suivantes :

- par transfert de type Southern des produits amplifiés sur membranes de nylon, suivi d'une révélation après hybridation avec une ou plusieurs sondes nucléotidiques spécifiques d'une séquence interne au produit amplifié (sondes de SEQ ID NO:22-37, notamment) marquées avec un isotope radioactif ou un fluorochrome, ; de manière plus précise, cette méthode comprend le transfert de type Southern sur membrane de nylon ou de nitrate de cellulose par capillarité buvard, par exemple avec un tampon 20X SSC, cross Linkage de l'ADNg sur la membrane après exposition aux UV 700 kjoules (étape bien connue dans le domaine de l'art), préhybridation avec du milieu d'hybridation dans des conditions permettant de diminuer le bruit de fond non spécifique, hybridation avec une ou plusieurs sondes d'oligonucléotides internes marquées radioactivement (méthode T4 kinase + $^{32}$PgammaATP) permettant de révéler de manière spécifique les produits de PCR et validation de la spécificité des réarrangements en mesurant la taille des produits de PCR. La quantification relative de l'intensité de chaque bande d'ADN correspondant aux différents réarrangements en utilisant un imageur et un logiciel adapté comme le Quantity-One de la société Biorad.
- par utilisation d'une base marquée (par un isotope radioactif ou un fluorochrome) lors de l'amplification puis par mesure de son incorporation directement dans le gel,
- par utilisation d'agent marquant l'ADN (tels que bromure d'éthidium, SybrGreen I ou autres) lors de la migration, et détection après excitation aux UV ou autre longueur d'onde appropriée
- par utilisation d'oligonucléotides marqués par des fluorochromes ou autres moyens de révélation enzymatiques (ex. Avidine-biotine, peroxydase...) lors de l'amplification.

Tout autre méthode de détection peut également être envisagée.

Selon une disposition avantageuse de ce mode de mise en oeuvre, les sondes sont avantageusement sélectionnées dans le groupe constitué par les séquences SEQ ID NO:22-37, telles que définies dans le Tableau II.

Un réarrangement donné est défini à partir des produits d'amplification : d'une part, d'après la distance de leur migration et d'autre part, par hybridation avec des sondes internes spécifiques. Il est possible de détecter individuellement plusieurs réarrangements dans une seule réaction. En combinant un nombre suffisant de réactions, il est possible de quantifier l'ensemble des réarrangements V(D)J constituant le répertoire immunitaire et ceci, directement au niveau de l'ADN génomique.

Avantageusement, le procédé décrit peut être mis en oeuvre sur différents types d'échantillons biologiques :

cellules en culture, lignées cellulaires, échantillons provenant de biopsies, de microdissection, de prélèvements sanguins, de cellules triées ; de préférence sur des prélèvements sanguins.

Le procédé décrit, tel que défini ci-dessus, permet notamment l'analyse totale ou partielle du répertoire immunitaire par l'analyse des combinaisons V(D)J, notamment par réalisation d'étapes (b) d'amplification en parallèle avec différents couples d'amorces. Cette méthode est à la fois qualitative et quantitative. La détection des recombinaisons génétiques V(D)J permet de suivre le répertoire immunitaire spécifique et d'en estimer sa diversité de manière dynamique dans différentes conditions pathologiques et suite à différents traitements. Basée sur le principe de l'amplification de l'ADN, cette technique permet de détecter de manière précoce et avec une faible quantité de matériel biologique, le répertoire immunitaire.

La mesure de ces répertoires constitue un défi difficile au vu du grand nombre de combinaisons du répertoire V(D)J. De plus, chaque récepteur est constitué de deux chaînes, ainsi le répertoire total pour un récepteur donné est le produit de la diversité structurale de chacune des chaînes le constituant. Les nombres de formes possibles de récepteurs Ig ou TCR sont estimés être de l'ordre de $10^{14}$ à $10^{18}$ récepteurs potentiels différents. Cependant, l'évaluation des répertoires demeure un enjeu d'importance pour évaluer la réponse immunitaire spécifique dans les conditions physio-pathologiques telles que les réponses aux infections, au développement de cancer, aux allergies, aux maladies auto-immunes, aux déficits immunitaires et à leur reconstruction,...

D'une part, on peut considérer que la diversité du répertoire immunitaire est en relation avec la santé de l'organisme. Un répertoire faiblement diversifié peut correspondre à un déficit immunitaire rendant l'organisme sensible à différentes pathologies. Dans un certain nombre de domaines médicaux et cliniques, il est important de suivre les variations des répertoires. On peut citer les situations de lymphopénies héréditaires (déficits immunitaires congénitaux) ou infectieuses (le SIDA par exemple), ou provoquées comme dans le cas des ablations du système immunitaire par irradiation corporelle dans le cas des traitements de cancers du sang (leucémies, lymphomes, etc).

D'autre part, un certain nombre d'essais thérapeutiques visent à induire une réponse immunitaire spécifique. C'est le cas par exemple des divers protocoles de vaccinations anti-microbiennes et anti-tumorales, utilisant diverses voies d'injections, de formulation, d'adjuvants, de différents vecteurs, etc. Dans d'autres cas, c'est une répression de la réponse immunitaire qui est recherchée comme par exemple, pour contrecarrer les syndromes auto-immuns et lutter contre les manifestations allergiques.

Dans tous ces cas, le procédé décrit permet d'une part de mesurer le répertoire des récepteurs des antigènes au cours des différentes phases des maladies, et d'autre part d'évaluer l'action stimulante ou répressive de molécules dans l'objectif d'en dériver des médicaments modulateurs de la réponse immunitaire spécifique.

En conséquence, sont également décrits :

* un procédé de suivi d'un traitement d'une pathologie dans laquelle le répertoire immunitaire est initialement modifié, chez un sujet concerné, lequel procédé est **caractérisé** :

- **en ce qu**'il met en oeuvre le procédé d'évaluation du répertoire immunitaire, tel que défini ci-dessus en début de traitement et
- en ce que ledit procédé d'évaluation est réitéré à différentes phases du traitement et
- en ce que le profil du répertoire immunitaire obtenu à chaque fois est comparé à celui d'un répertoire immunitaire standard, et ce afin d'évaluer la réponse dudit individu audit traitement.

* un procédé de mesure du répertoire des récepteurs des antigènes au cours des différentes phases d'une pathologie dans laquelle le répertoire immunitaire est modifié, chez un sujet concerné, lequel procédé est **caractérisé :**

- **en ce qu**'il met en oeuvre le procédé d'évaluation du répertoire immunitaire, tel que défini ci-dessus à différentes phases de la pathologie et
- en ce que le profil du répertoire immunitaire obtenu à chaque fois est comparé à celui d'un répertoire immunitaire standard, et ce afin d'évaluer l'évolution de ladite pathologie.

Conformément à la divulgation, l'échantillon biologique est constitué de lymphocytes T de n'importe quelle origine ; plus précisément, l'échantillon biologique peut être constitué de cellules thymiques, de lymphocytes T du sang périphérique ou d'autres organes lymphoïdes, de lymphocytes T provenant d'autres tissus et en particulier de lymphocytes T issus des tumeurs, de sites inflammatoires, ou de divers organes tels que intestin, poumon, foie etc...

Est décrit en outre un kit d'évaluation quantitative du répertoire immunitaire d'un individu, **caractérisé en ce qu**'il comprend outre les tampons et réactifs usuels pour mettre en oeuvre une PCR, les amorces et les sondes telles que définies ci-dessus.

Les pathologies concernées sont aussi bien des tumeurs des lymphocytes et cellules apparentées aux lymphocytes que toute autre pathologie impliquant la réponse immunitaire, telles que des maladies virales, des maladies auto-immunes, des états physiopathologiques, des immunodéficiences, des allergies ou des anomalies des mécanismes de recombinaison V(D)J.

Sont décrites également des amorces susceptibles d'être mises en oeuvre dans un procédé tel que défini ci-dessus, **caractérisées en ce qu**'elles sont sélectionnées dans le groupe constitué par les amorces oligonucléotidiques répondant aux séquences SEQ ID NO :1-21.

Sont décrites en outre des sondes de détection susceptibles d'être mises en oeuvre dans un procédé tel que défini ci-dessus, **caractérisées en ce qu**'elles sont sélectionnées dans le groupe constitué par les sondes oligonucléotidiques de séquences SEQ ID NO:22-37.

Est décrite également l'utilisation des amorces d'amplification et des sondes de détection telles que définies ci-dessus pour l'évaluation quantitative du répertoire immunitaire d'un individu.

Outre les dispositions qui précèdent, encore d'autres dispositions ressortiront de la description qui va suivre, qui se réfère à des exemples de mise en oeuvre du procédé décrit ainsi qu'aux dessins annexés, dans lesquels :

- la figure 1 représente les résultats d'un procédé décrit (longue PCR multiplex) effectué pour évaluer le réarrangement V8-Jα dans le thymus : analyse globale de la région Jα. Les réactions PCR multiplex sont mises en oeuvre sur l'ADNg en utilisant les amorces Vα de la famille hV8 (voir Tableau I) en combinaison avec 11 amorces Jα différentes (voir Tableau I : SEQ ID NO :11-21). Les produits sont analysés par Southern blot à l'aide de sondes spécifiques de la famille V8 (SEQ ID NO:24). Chaque piste correspond à une réaction PCR individuelle ; chaque bande correspondant à un réarrangement avec le segment Jα indiqué sur la gauche de la piste. Compte tenu du fait que l'amplification par PCR est plus efficace sur des produits de petite taille que sur des produits de grande taille, les bandes situées à la base de chaque piste sont plus intenses. Les astérisques indiquent les produits non-spécifiques déterminés par la distance de migration. Représentation de la région J (pas à l'échelle) : les 11 amorces choisies sont réparties sur la région Jα comme indiqué. Les points indiquent la position des amorces et des sondes Jα ; les lignes correspondantes indiquent l'étendue des segments Jα détectables dans chaque piste. Les lignes en pointillés indiquent les pseudogènes Jα non transcrits.
- la figure 2 illustre le rôle de la localisation des gènes Vα dans la répartition du réarrangement avec la région Jα. Les réarrangements V-Jα sont analysés par PCR multiplexe sur de l'ADN extrait à partir de thymus total de nourrissons de 6 jours. Les amorces spécifiques des gènes Vα sont indiquées et correspondent aux amorces décrites dans le Tableau ci-dessus ; ces amorces sont utilisées avec 7 amorces J indiquées au dessus de chaque piste (voir également les séquences dans le Tableau I ci-dessus).
- la figure 3 illustre une représentation de la matrice du répertoire humain V-Jα, le réarrangement dépendant de la position desdits gènes sur le locus. Figure 3a : panneau représentatif de la quantification relative d'un réarrangement V-Jα, par intégration des signaux des produits PCR de la figure 2. Cette intégration est réalisée sur les produits de PCR dont la réaction PCR est arrêtée pendant la phase exponentielle et après vérification que l'intégration est linéaire. L'axe des Z illustre l'intensité de signal relatif des réarrangements détectés ; l'axe des J représente l'ordre relatif des segments Jα étudiés ; les gènes Vα étudiés sont indiqués sur l'axe V. Les flèches illustrent les tendances de progression des réarrangements Vα-Jα. Figure 3b gauche : analyse de l'utilisation de la région Vα globale par intégration des 7 segments Jα étudiés à la figure 3a ; figure 3b droite : analyse de l'utilisation de la région Jα globale par intégration des 10 gènes Vα étudiés à la figure 3a.
- la figure 4 illustre le principe du procédé décrit, appliqué à la détection des recombinaison V8-J : les amorces mises en oeuvre sont les suivantes : SEQ ID NO :5 (hTRAV8) et SEQ ID NO :13 (hTRAJ41); la sonde de détection est la sonde hAJ41p (SEQ ID NO:29).
- la figure 5 représente une comparaison entre le score RSS des gènes Vα humains, la position dans le locus et le segment J utilisé principalement.
- la figure 6 représente un profil de réarrangement de la chaîne TCRα humaine dans les lymphocytes du sang périphérique. Un exemple du profil de réarrangement TCRα d'un donneur est représenté (PCR multiplex). Six oligonucléotides spécifiques de Vα ont été sélectionnés et utilisés en combinaison avec neuf amorces spécifiques de Jα (voir au-dessus de chaque piste). Cette analyse a été effectuée avec trois ADNs additionnels obtenus à partir de lymphocytes de sang périphérique d'individus sains (âge 25-55) et des résultats comparables ont été obtenus. Les analyses PCR multiplex ont été reproduites plusieurs fois pour chaque ADN.
- la figure 7 illustre l'abondance relative des réarrangements V-Jα spécifiques chez six sujets sains, déterminée par analyse PCR génomique quantitative. Les réarrangements sélectionnés sont les suivants : Vα1, 40, 41 et Jα56, 53, 41, 33, 10. Pour chaque réarrangement, le cycle, au cours duquel un produit est détecté, est indiqué. Trois ADN de thymus (A) provenant respectivement d'un enfant âgé de 6 jours, de 10 jours et de 3 mois ainsi que des lymphocytes du sang périphérique (PBL) (B) provenant de six sujets sains âgés de 25 à 55 ans ont été analysés. La normalisation du contenu en ADN de chaque échantillon est réalisée par amplification du gène domestique G3PDH. Les données présentées correspondent à celles obtenues à partir de trois expériences différentes qui ont conduit à des résultats similaires. Les résultats sont exprimés en nombre de cycles nécessaires à l'apparition du produit lié à un réarrangement spécifique chez les différents sujets. Un nombre de cycles peu important implique la présence de quantités d'ADN plus importantes et ainsi plus de recombinaisons.

- la figure 8 représente :

  en (A), la quantification des transcrits de TCRα: l'ADNc, extrait à partir d'échantillons de lymphocytes du sang périphérique obtenus à partir de 10 sujets sains, a été amplifié en utilisant des oligonucléotides de différentes familles ADV en combinaison avec une amorce spécifique du fragment constant de la chaîne α (amorce AC). La courbe d'amplification illustrée à la figure 8(A) est représentative de l'un des 10 échantillons analysés. Les résultats sont représentés pour ADV1, 3, 5, 6, 7, 8, 10, 13, 16, 17, 19, 20, 22, 25, 26, 27, 30, 36, 38, 40, DV3. Les courbes correspondent au log de l'intensité de fluorescence en fonction du nombre de cycles.

  en (B), l'abondance relative des transcrits ADV-AC par PCR quantitative en temps réel: les familles ADV sont indiquées uniquement par leur nombre, en relation avec le cycle au cours duquel le produit est détecté.

Une comparaison entre quatre ADNc de lymphocytes du sang périphérique et un ADNc de thymus d'un enfant de 3 mois a été établie.

On observe une fréquence similaire de l'expression des familles Vα dans les autres échantillons, les résultats obtenus étant reproductibles.

Il doit être bien entendu, toutefois, que ces exemples sont donnés uniquement à titre d'illustration de l'objet de l'invention, dont ils ne constituent en aucune manière une limitation.

**EXEMPLE 1 : Matériel et méthodes**

- Nomenclature :

Les nomenclatures pour les gènes TRAV (Vα) et les segments TRAJ (Jα) sont conformes à celles de la base IMGT (http://imgt.cines.fr). La carte détaillée des locus TCRAD humain et de souris sont accessibles sur le site IMGT à l'adresse suivante :

http://imgt.cines.fr/textes/IMGTrepertoire/LocusGenes/locus/human/TRA/Hu_TRAm ap.html.

Les séquences utilisées pour établir chaque amorce ont été extraites à partir de la base de données Genbank n° d'accès NG_001332.

- Extraction d'ADNg

Un ensemble de précautions est à suivre pour préserver l'intégrité de l'ADN génomique (ADNg) notamment la taille, la pureté, l'absence de contamination en RNA et en sel. Le rendement d'amplification par PCR de long brin d'ADN est conditionné par ces critères. Globalement, il faut respecter les précautions définies pour la purification de l'ADNg utilisé pour la technique de transfert de type Southern (voir Sambrook J., Fritsch EF., Maniatis T. 1989. Molecular cloning. A laboratory manual. Second Edition. Cold Spring Harbor, New York, EUA).

- Il faut éviter de vortexer l'ADNg, plus on le vortex et plus il se casse.
- Il faut préserver l'ADNg dans un tampon légèrement basique (ex : TRIS 10 mM ; pH entre 7,5 et 8,5)
- Il faut éviter de faire des pipetages brusques (casse l'ADN au niveau de la pointe du cône)
- L'ADNg est conservé à 4°C pour éviter sa dégradation
- En cas de conservation prolongée, mettre l'ADNg dans de l'alcool.

Il est préconisé un Kit d'extraction Quiagen : Genomic-Tip System. http://www1.qiapen.com/Products/GenomicDnaStabilizationPurification/QiapenGeno micTipSystem/

Ce Kit permet de préserver des fragments d'ADN de l'ordre de 150 Kb.

Le type de protocole d'extraction utilisé par le Kit peut être employé pour travailler sur différentes sources de cellules comme par exemple le sang, la culture cellulaire, des cellules issues de tri cellulaire, des tissus (thymus, ganglions lymphatiques... liste non exhaustive). Dans le cas de tissus, avant de traiter avec le kit, 1) pour les tissus peu cohésifs (thymus, ganglions, ...) on tente d'individualiser les cellules par un traitement modéré à la trypsine - EDTA, 2) pour les tissus hautement cohésifs (type muscle par exemple) on réduit en poudre le tissus par un traitement avec un pilon à froid en présence de neige carbonique.

- Echantillons d'ADN :

* Thymus

L'ADN génomique humain de 3 thymus entiers, (un nourrisson de sexe féminin de 6 jours, deux nourrissons de sexe masculin de 10 et 90 jours) est extrait et amplifié comme décrit dans Gallagher et al. (28), la PCR multiplex et le Southern blot sont réalisés comme décrits dans Mancini et al. (27).

* Cellules mononucléées du sang périphérique (PBMC)

Les cellules sont séparées par gradient de densité de Ficoll. Les échantillons proviennent de sujets sains âgés de 25 à 55 ans. Les ADNc sont extraits comme décrit dans Pernollet M. et al..

- PCR multiplex :

Brièvement, la PCR multiplex s'effectue à l'aide d'amorces situées en amont de V et en aval de J (voir Tableau I ci-dessus) qui permettent d'amplifier jusqu'à 10 kb. Après chaque réaction, la spécificité des produits de PCR est vérifiée par une hybridation de sondes V (SEQ ID NO:22-26) et J internes (SEQ ID NO :27-37) (voir Tableau II ci-dessus) et par analyse informatique de la distance de migration (Quantity One 4.2.1 Software-Biorad, France).

Les amplifications sont réalisées avec 1,3 unité par réaction de "expend high fidelity PCR system" (Roche Diagnostics, Meylan, France) dans les conditions suivantes :

- Dénaturation initiale 1-2 minutes à 94°C
- 15 cycles successifs comprenant : une étape de dénaturation : 5 à 30 secondes à 90-94°C, une étape d'hybridation des amorces : 15-30 secondes à 58-62°C et une étape d'élongation : 14 à 20 minutes à 68-72°C et
- 15 cycles successifs comprenant : une étape de dénaturation : 5 à 30 secondes à 90-94°C, une étape d'hybridation des amorces : 15-30 secondes à 58-62°C et une étape d'élongation : 14 à 20 minutes à 68-72°C + 15-20 secondes additionnelles à chaque cycle supplémentaire et
- 1 cycle final d'élongation de 10 minutes à 72°C.

Alternativement, les conditions peuvent également être les suivantes :

- dénaturation initiale de 5 min à 94°C,
- 26 cycles successifs comprenant une étape de dénaturation : 30 secondes à 94°C ; une étape d'hybridation des amorces : 30 secondes à 58°C et une étape d'élongation : 10 minutes à 72°C et
- un cycle final d'élongation de 10 minutes à 72°C.

La normalisation de la quantité d'ADN dans chaque réaction est déterminée par amplification d'un gène non soumis à réarrangement dans les mêmes cycles de PCR. L'efficacité des amorces est vérifiée par dilution successive de la matrice (28).

Toutes les amorces sélectionnées (voir Tableau 1 : SEQ ID NO :1-21) présentent une efficacité d'amplification de 98 % permettant une comparaison relative directe.

La séparation des fragments d'ADN se fait par migration électrophorétique (gel d'agarose, de polyacrylamide, séparation en microcapillaires sur bioanalyseur AGILENT ou encore migration en champ pulsé).

La révélation peut être réalisée par les techniques suivantes qui ne sont pas limitatives : i) transfert de type Southern sur membrane de nylon et hybridation de sondes marquées (radio isotopes ou fluorochromes), ii) utilisation d'une base marquée lors de l'amplification et mesure de son incorporation directement dans le gel, iii) utilisation d'un agent marquant l'ADN (BET ou Cybergreen) lors de l'amplification ou encore iv) utilisation lors de l'amplification, d'oligonucléotides marqués par des fluorochromes ou autres moyens de révélation enzymatique (avidine-biotine, peroxydase).

- Conception des amorces :

Les amorces spécifiques des segments de gènes Vα et Jα du locus TCRAD humain ont été sélectionnées pour leur spécificité de séquences, comme précisé ci-dessus, à l'aide du logiciel NTI Vector-8suite, Informax. L'hybridation non spécifique est vérifiée sur Blast dans le site www.ensembl.org. Les amorces sélectionnées correspondent à celles illustrées au Tableau I ci-dessus.

Plus précisément :

. **pour les gènes V** : au niveau génomique, on retrouve les éléments correspondant à une région régulatrice de type promoteur avant le gène, une région codant le peptide signal ou « leader » (Exon 1), un intron et une région codant la région variable (Exon 2) et une région RSS non traduite assurant la spécificité spatiale des réarrangements. Ainsi, l'amorce variable (V) est choisie pour s'hybrider à une région codante ou non, mais dans tous les cas en amont de la séquence RSS du gène V.

. **pour les gènes J** : l'amorce est choisie en aval (correspondant au 3' du brin transcrit) du gène J à étudier ; il peut être dans la région codante du gène J ou dans une région non codante entre deux gènes J.

- PCR quantitative en temps réel d'ADNg :

Les réactions de PCR sont réalisées sur un cycleur Light mettant en oeuvre le kit FastStart® à une unité/réaction (Roche Diagnostics).

50 ng d'ADN est utilisé pour chaque réaction et la quantité d'ADN entre les échantillons est normalisée par amplification du gène domestique G3PDH.

Les conditions d'amplification pour les échantillons d'ADN sont les suivants : 94°C 10 minutes, puis 41 cycles (94°C 15 sec, 67°C 7 sec, 72°C 7 sec).

La spécificité du produit unique d'amplification est déterminée par analyse de la courbe de fusion en accord avec les instructions du fabricant (Roche Diagnostics) et par migration sur gels d'agarose et détermination de la taille d'un réarrangement spécifique.

Chaque échantillon est analysé en triple, dans trois essais distincts.

Les résultats sont exprimés en nombre de cycles (abondance relative) pour un réarrangement spécifique dans les différents échantillons d'ADN de thymus et de lymphocytes de sang périphérique analysés.

- PCR quantitative en temps réel d'ADNc :

L'ARN de thymus (enfants de 10 j et de 3 mois) et de 10 échantillons de lymphocytes du sang périphérique est isolé en utilisant le kit d'isolement d'ARN RNeasy (Quiagen), en suivant les instructions du fabricant.

La transcription inverse est réalisée à l'aide du kit SuperScript II RNase $H^-$ (Life Technologies), conformément au protocole suggéré par le fabricant.

Les ADNc de différentes réactions de synthèse sont mélangés et le même échantillon est utilisé pour toutes les PCRs.

Des dilutions appropriées de chaque échantillon sont sélectionnées pour fournir des taux équivalents de produit par normalisation avec le gène domestique G3PDH et le gène CD3.

Les conditions d'amplification des échantillons d'ADNc sont les suivantes : 95°C 10 min, puis 47 cycles (95°C 15 sec, 70°C 10 sec, 72°C 15 sec). Pour amplifier spécifiquement un maximum de membres de la famille du gène Vα dans les mêmes conditions de PCR, la température d'hybridation a été augmentée à des conditions plus stringentes.

Selon les instructions du fabricant (Roche Molecular Biochemicals), l'efficacité d'amplification pour une réaction de PCR donnée est calculée comme suit :

$$E = 10^{-1/\text{pente}}.$$

Le maximum d'efficacité possible pour une PCR est E = 2 : chaque produit de PCR est répliqué à chaque cycle et correspond à une pente de -3,3 ($2=10^{-1/-3,3}$).

Dans les conditions expérimentales sélectionnées, toutes les réactions de PCR V-Cα présentent des pentes similaires de courbes d'amplification, de 3,67 à 3,73, indiquant des efficacités de réaction comparables et fournissant un rendement moyen pour les réactions ADV-AC de 85-87 %.

La spécificité du produit unique d'amplification est établie par analyse de la courbe de fusion, par migration en gels d'agarose et détermination de la taille d'un réarrangement spécifique et par séquençage.

Les courbes de fusion des produits de PCR sont déterminées selon les instructions du fabricant (Roche Diagnostics). Chaque échantillon est analysé en triplicat, dans deux essais distincts.

**EXEMPLE 2: Analyse de la région J humaine comparativement à son homologue murin (thymus)**

Des PCR multiplex (conditions de l'exemple 1) sont réalisées en combinant des amorces d'un gène V individuel et 11 amorces différentes en aval de AJ (J1, J5, J10, J18, J24, J29, J33, J41, J48, J53, J56) couvrant la région J, comme représenté sur la figure 1. Chaque colonne correspond à une réaction de PCR individuelle et chaque bande correspond à un réarrangement de V avec un segment J, lequel est indiqué en chiffre à gauche de chaque piste.

**Détermination du nombre de segments J potentiels utilisés dans les réarrangements V-J.**

Trois des six membres de la famille V8 humaine (V8.2, V8.4 et V8.6) respectivement situé à 701, 653, et 569 kb du gène Cα, sont amplifiés par la technique de PCR multiplex décrite ci-dessus, à l'aide des amorces précédemment définies.

Les résultats représentés sur la figure 1 montrent que les membres V8 humains réarrangent avec les segments J allant de Jα61 à Jα3. La totalité de la région J est donc accessible à la recombinaison.

**Détermination des segments de gènes non fonctionnels.**

Les segments de gènes non fonctionnels des loci de TCR sont également appelés "pseudogènes de réarrangement ". Le procédé selon la présente invention permet de caractériser ces pseudogènes. Aussi bien chez la souris que chez l'homme certains segments ont été identifiés comme n'étant pas fonctionnels (pseudogènes).

Chez la souris, 16 gènes Jα sur 60 sont des pseudogènes (24, 29), tandis que chez l'homme jusqu'à présent seulement les pseudogènes Jα51, 59 et 60 avaient été caractérisés (25).

Comme le montre la figure 1, cinq segments Jα humains supplémentaires sont incapables de donner un réarrangement avec des membres de la famille V8. Ces cinq segments Jα sont : Jα55, Jα25, Jα19, Jα2 et Jα1. On observe la même chose avec d'autres familles V (figure 2).

Par ailleurs, les gènes de non réarrangement Jα46, 41, 36, 29, 20, 14, 8 et 3 décrits chez la souris sont fonctionnels chez l'homme. La région Jα humaine contient plus de segments Jα fonctionnels, capables de réarrangements, que la souris, ce qui implique un répertoire J plus diversifié.

**EXEMPLE 3 : Analyse qualitative des réarrangements V-J** humains

Quatre gènes Vα les plus distants de la région Jα (V1, V2, V3 et V5) et cinq gènes Vα proximaux à la région Jα (V26.2, V35, V38, V40, V41, situés entre - 345 et -227 kb par rapport au gène Cα) ont plus particulièrement été étudiés.

La famille multigénique V8 est utilisée comme contrôle d'utilisation de la région J du fait qu'elle est située au milieu du locus et qu'elle est composée des membres situés à -701, -653, -569 kb de la région Cα.

Des amorces spécifiques des gènes V (voir Tableau I) sont utilisées en combinaison avec 7 amorces spécifiques de la région Jα (53, 48, 41, 29, 18, 10 et 5) (figure 2). Pour faciliter l'analyse, un seul gène V à la fois est soumis au réarrangement avec la région Jα. L'étude est réalisée dans les conditions de PCR multiplex de l'exemple 1 (voir également la figure 4).

La figure 2 représente le profil de réarrangement des gènes V pris individuellement avec la région Jα. Celle-ci permet de constater que les gènes V proximaux par rapport à la région Jα se réarrangent principalement avec les gènes J en 5' les plus proximaux (V26.2, V 35, V38, V40 et V41 avec les segments Jα53, Jα48, Jα41, Jα29).

Ces résultats montrent également que les gènes V distaux par rapport à la région Jα se réarrangent principalement avec les segments Jα situés au centre et dans la partie 3' de cette région.

Le gène V8 contrôle permet de vérifier que la recombinaison est homogène quelle que soit la région Jα. La figure 4 montre les recombinaisons obtenues avec les amorces SEQ ID NO:5 et 13 et la sonde de détection SEQ ID NO :29.

Les réarrangements V-J sont donc dépendants de la localisation des segments sur les chromosomes et chaque gène V se réarrange avec une quantité restreinte de segments J.

**EXEMPLE 4 : Analyse quantitative des réarrangements V-J humains**

Les réarrangements V-J peuvent, dans les conditions de l'exemple 1, être analysés de façon quantitative à l'aide d'une quantification relative de l'intensité de chaque bande d'ADN correspondants aux différents réarrangements, en utilisant un imageur et un logiciel adapté tel que le Quantity-One (Biorad).

La figure 3a présente une représentation matricielle du répertoire V-Jα humain. L'axe Z présente l'intégration de l'intensité relative des réarrangements détectés, l'axe J représente l'ordre d'analyse des segments et l'axe V représente les gènes V étudiés allant de la zone proximal à la zone distale.

On constate sur cette figure que les gènes situés entre V41 et V26.2 (situé à 345 kb du locus TCRD) réarrangent 3 à 7 fois plus que ceux compris entre V5 et V1 situés respectivement à 798 et 925 kb. Concernant la région J, les gènes en 5' les plus proximaux se réarrangent 3 à 7 fois plus que les gènes distaux. Enfin, la famille multigénique V8 se combine à fréquence égale avec l'ensemble des segments de gènes J, quelle que soit leur position, confirmant l'hypothèse que les réarrangements sont fonction de la position des gènes sur le locus.

La figure 3b représente pour les gènes V (à gauche) et les gènes J (à droite) l'utilisation globale de chacun des segments.

**EXEMPLE 5 : Répertoire TCRα des lymphocytes T périphériques humains.**

Pour déterminer les profils de recombinaison des gènes TCRα des lymphocytes T périphériques, une approche similaire à celle mise en oeuvre pour le thymus est utilisée (voir exemple 2).
Six gènes de la famille Vα ont été sélectionnés (TRAV) répartis dans la région distale du locus (V1 et V2), dans la région centrale du locus (V8) et dans la région proximale du locus (V38, V40 et V41).
Leurs profils de réarrangement en utilisant neuf amorces Jα ont été étudiés.
L'analyse a été réalisée sur quatre échantillons indépendants, provenant de quatre individus sains (de 25 à 55 ans).
Les résultats obtenus indiquent que les profils de recombinaison du locus TCRAD sont remarquablement similaires chez les différents individus sains.

TABLEAU IV

| Données de la PCR multiplex sur quatre échantillons d'ADNs obtenus à partir de lymphocytes du sang périphérique de sujets sains (PBL1 à PBL4) | | | | | | | | | | | |
|---|---|---|---|---|---|---|---|---|---|---|---|
| ***V/J*** | | ***56*** | ***53*** | ***48*** | ***41*** | ***33*** | ***29*** | ***24*** | ***18*** | ***5*** | ***1*** |
| ***V1*** | ***PBL 1*** | - | - | ***ND*** | - | + | + | + | + | - | + |
| | ***PBL 2*** | - | - | - | + | + | + | + | + | - | + |
| | ***PBL 3*** | - | - | ***ND*** | - | + | + | + | + | - | + |
| | ***PBL 4*** | - | - | - | + | + | + | + | + | - | + |
| | | | | | | | | | | | |
| ***V2*** | ***PBL 1*** | - | - | ***ND*** | + | + | + | + | + | + | - |
| | ***PBL 2*** | - | - | - | + | + | + | + | + | + | - |
| | ***PBL 4*** | - | - | + | - | + | - | ***ND*** | - | - | + |
| | | | | | | | | | | | |
| ***V8*** | ***PBL 1*** | + | + | + | + | + | + | + | + | + | + |
| | ***PBL 2*** | + | + | + | + | + | + | + | + | + | + |
| | ***PBL 3*** | + | + | + | + | + | + | + | + | + | + |
| | ***PBL 4*** | + | + | + | + | + | + | + | + | + | + |
| | | | | | | | | | | | |
| ***V38*** | ***PBL 1*** | + | + | + | + | + | + | ***ND*** | + | - | + |
| | ***PBL 3*** | + | + | + | + | + | + | + | - | - | - |

(suite)

| Données de la PCR multiplex sur quatre échantillons d'ADNs obtenus à partir de lymphocytes du sang périphérique de sujets sains (PBL1 à PBL4) | | | | | | | | | | | |
|---|---|---|---|---|---|---|---|---|---|---|---|
| | **PBL 4** | + | + | + | + | + | + | **ND** | + | - | - |
| | | | | | | | | | | | |
| **V41** | **PBL1** | + | + | - | + | + | + | **ND** | - | - | - |
| | **PBL 2** | + | - | **ND** | + | - | + | +/- | + | - | - |
| | **PBL 3** | + | - | **ND** | + | - | +/- | +/- | + | - | - |
| | **PBL 4** | + | - | **ND** | + | - | + | - | + | - | - |
| | | | | | | | | | | | |
| **V40** | **PBL 1** | + | + | - | + | + | + | **ND** | - | - | - |
| | **PBL 2** | - | + | **ND** | + | + | +/- | + | - | - | - |
| | **PBL 4** | + | + | + | + | + | +/- | **ND** | - | - | - |

Les résultats obtenus montrent que la « règle » de proximité observée dans le thymus s'applique également.

Le profil de réarrangement d'un échantillon de lymphocytes (parmi les quatre échantillons analysés) est représenté à la figure 6.

En particulier, les gènes Vα les plus proximaux (V38, V40 et V41) sont principalement réarrangés avec les gènes Jα proximaux.

La famille multigénique V8, localisée au milieu du locus Vα est réarrangée de manière égale avec tous les segments Jα du locus.

Les gènes Vα distaux (V1 et V2) sont réarrangés de préférence avec la partie centrale et 3' ainsi qu'avec les gènes Jα les plus près.

Chez certains individus, on observe des différences discrètes dans leurs profils combinatoires (voir Tableau V). Par exemple, les réarrangements impliquant V1-J41, V2-J41 et V2 avec les gènes J les plus éloignés ou pour les Vα les plus proximaux, les réarrangements impliquant V40-J56, J29 et V41-J53, J24, ne sont pas toujours détectés, en comparaison avec le thymus.

Cependant, ces différences n'affectent pas les règles combinatoires générales et confirment la similarité des profils entre individus sains.

- <u>Analyse des fréquences des réarrangements spécifiques dans différents échantillons d'ADN de lymphocytes T périphériques humains :</u>

Alors que les profils de recombinaison sont similaires dans le thymus et les lymphocytes périphériques, les profils PCR multiplex montrent des fréquences de réarrangement différentes pour des combinaisons spécifiques.

Pour analyser ces différences de manière plus précise, une PCR quantitative en temps réel effectuée à partir d'ADNg a été développée.

Les réarrangements ont été analysés sur de l'ADN obtenu à partir de lymphocytes périphériques de six sujets sains, incluant les trois des quatre échantillons testés en PCR multiplex, ainsi que les trois ADN thymiques testés en PCR multiplex.

Ont été plus particulièrement étudiés, les réarrangements V1, V40 et V41 en combinaison avec les gènes proximaux J56 et J53, les gènes centraux J41 et J33 et le gène distal J10.

Les résultats sont illustrés à la figure 7 et sont exprimés en nombre de cycles pour l'apparition du produit de chaque réarrangement, le premier produit à apparaître étant le plus abondant.

A partir de ces résultats, les observations suivantes peuvent être faites :

- quelques combinaisons V-Jα ne sont pas détectables par les analyses PCR effectuées, sans doute en raison du fait qu'elles sont peu fréquentes (V1-J56, V1-J53, V40-J10 et V41-J10). Ce résultat confirme le profil combinatoire déjà observé (voir figures 2 et 6) qui dépend des positions réciproques des gènes Vα et Jα dans le locus et implique, pour tous les ADNs testés, une combinaison Vα proximal-Jα proximal et Vα distal-Jα distal.
- les réarrangements sont quantitativement légèrement moins importants dans les cellules de la périphérie par rapport au thymus (figures 7A et 7B).

En particulier, les réarrangements proximaux V-Jα, tels que V40-J56 ou V41-J56 et V41-, V40-J53 sont faiblement trouvés dans l'ADN des lymphocytes testés (8 à 64 fois moins important), par rapport à ce que l'on trouve dans le thymus.

Ces différences peuvent être expliquées de plusieurs manières : 1) le nombre différent de cellules T entre le thymus et le sang périphérique ; 2) la contribution des réarrangements sur les cercles d'excision qui pourrait être amplifiée dans le thymus, est diluée dans les cellules T périphériques ; 3) l'occurrence de réarrangements secondaires dans le thymus ou des évènements de révision des récepteurs à la périphérie, qui pourrait remplacer les réarrangements proximaux par la jonction des segments V-Jα plus distaux ; 4) des évènements de sélection négative.

- quelques combinaisons, telles que V1-J33 sont favorisées à la périphérie (haute fréquence = nombre de cycles faible) chez tous les individus testés.
- On peut également identifier des expansions/contractions de réarrangements spécifiques chez certains individus : V40-J41, V1-J41, V1-J10, V41-J41, V41-J33, V40-J33.

D'autres réarrangements, tels que V41-J41 ne sont pas retrouvés chez certains individus et reflètent vraisemblablement un événement de sélection négative.

Ces résultats montrent qu'alors que le profil de recombinaison est quantitativement similaire parmi les différents échantillons de thymus, on observe une plus grande hétérogénéité parmi les échantillons de lymphocytes T périphériques.

Cette divergence parmi les individus est reliée à différents évènements tels que la sélection thymique, les expansions de certains clonotypes induits par une réponse immune ou des forces de maintien de l'homéostasie.

- Fréquence des transcrits de la famille V chez plusieurs individus :

Des essais antérieurs (48), mettant en oeuvre une analyse semi-quantitative du gène Vα ont montré un biais en terme d'expression préférentielle de certains segments Vα sur les cellules CD4+ ou CD8+.

Cependant, une analyse fine de la prévalence de certaines familles Vα spécifiques n'a pas été établie en raison de l'absence de réactifs Vα spécifiques.

Pour surmonter ce problème, une analyse PCR quantitative en temps réel a été développée.

Des amorces spécifiques ont été sélectionnées (pour 26 sur les 34 familles Vα), ce qui permet une couverture d'environ 77 % des familles. L'efficacité obtenue, due au choix des amorces effectué, a permis de comparer les fréquences d'expression des familles Vα chez différents individus.

Le Tableau V représente les amorces mises en oeuvre pour mesurer la fréquence des transcrits de la famille V.

TABLEAU V

| Primer | Sequence 5'- 3' |
|---|---|
| hTRAV 1b | GCAACATGCTGGCGGAGCACCCAC(SEQ ID NO:46) |
| hTRAV 2b | ATGGCTTTGCAGAGCACTCTGG (SEQ ID NO:47) |
| hTRAV 3c | GCCTCTGCACCCATCTCGA (SEQ ID NO:48) |
| hTRAV 5b | GAGGATGTGGAGCAGAGTCTTTTC (SEQ ID NO:49) |
| hTRAV 6 | CGGCCACCCTGACCTGCAACTATA (SEQ ID NO:50) |
| hTRAV 7C | GGGACCCCAGCAGGGAGACGTTGCC(SEQ ID NO:51) |
| hTRAV 8-1 | ATGCTCCTGTTGCTCATACCAGTG (SEQ ID NO:52) |
| hTRAV 9-2 | CCTGAAAGCCACGAAGGCTGATGA(SEQ ID NO:53) |
| hTRAV 10C | GCATCTGACGACCTTCTTGGT (SEQ ID NO:54) |
| hTRAV 12-b | CCATGATGCGGGGACTGGAGTTGC (SEQ ID NO:55) |
| hTRAV 13-1 | CATTCGTTCAAATGTGGGCGAAAA(SEQ ID NO:56) |
| hTRAV 14c | CAGAAGATAACTCAAACCCAACCA(SEQ ID NO:57) |
| hTRAV 16b | AGAGTGACTCAGCCCGAGAAG(SEQ ID NO:58) |
| hTRAV 17 | CCGGGCAGCAGACACTGCTTCTTA (SEQ ID NO:59) |

(suite)

| Primer | Sequence 5'- 3' |
|---|---|
| hTRAV 19 | TCGTCGGAACTCTTTTGATGAGCA (SEQ ID NO:60) |
| hTRAV 20 | GTCTTGTGGCTTCAGCTTGGC (SEQ ID NO:61) |
| hTRAV 21 | TGCCTCGCTGGATAAATCATCAGG (SEQ ID NO:62) |
| hTRAV 22D | GGGAGCTCTGCTGGGGCTCTTGAG (SEQ ID NO:63) |
| hTRAV 24B | GCAGCTTCCCTTCCAGCAAT(SEQ ID NO:64) |
| hTRAV 25 | GGAGAGGACTTCACCACGTACTGC (SEQ ID NO:65) |
| hTRAV 26/DV7B | GGCTGGTGGCAAGAGTAACTG (SEQ ID NO:66) |
| hTRAV 27 | CACTGCGGCCCAGCCTGGTGATAC (SEQ ID NO:67) |
| hTRAV 29B | CAGCAAGTTAAGCAAAATTCACCA(SEQ ID NO:68) |
| hTRAV 30c | GCCGTGATCCTCCGAGAAGGGG (SEQ ID NO:69) |
| hTRAV 34 | TGATGATGCTACAGAAAGGTGGGG(SEQ ID NO:70) |
| hTRAV 35 | GGCTGGGAAGTTTGGTGATATAGTGTC (SEQ ID NO:71) |
| hTRAV 36/DV7 | ATGATGAAGTGTCCACAGGCT(SEQ ID NO:72) |
| hTRAV 38 | AGCAGCCAAATCCTTCAGTCTCAA(SEQ ID NO:73) |
| hTRAV 40 | AAGACAAAAACTCCCCCATTGTGAAATA(SEQ ID NO:74) |
| hTRDV 3 | CAGAGTTCCCCGGACCAGAC (SEQ ID NO:75) |

Les résultats de la PCR quantitative sont exprimés en terme de nombre de cycles pour l'apparition d'un produit donné pour chaque famille V-Cα.

L'ordre d'apparition des différents produits V-Cα correspond à leur abondance relative dans l'ADNc considéré de l'échantillon, le transcrit le plus abondant étant détecté en premier.

Une courbe d'amplification représentative d'un ADNc de lymphocytes T périphériques normaux, obtenus à partir de 10 individus, est représentée à la figure 8A.

Les données indiquent que les familles Vα humains ne sont pas exprimées au même taux dans des lymphocytes T lors de la sélection au niveau du thymus et dans des lymphocytes T périphériques matures ; ces données montrent également que la fréquence de l'expression de Vα chez des sujets d'espèces différentes et avec différents types de CMH, sont néanmoins similaires (figure 8B).

Par l'analyse des fréquences du gène Vα, on peut ainsi identifier un profil d'expression des familles ADV.

Si l'on considère qu'une différence maximum dans l'efficacité d'amplification est de 2 % par cycle parmi les différentes combinaisons V-Cα, sur par exemple 30 cycles d'amplification, ceci implique qu'il peut y avoir un cycle de différence dans la mesure des différents produits.

On peut ainsi définir plusieurs groupes :

. groupe 1 : apparition au $1^{er}$ ou au $2^{ème}$ cycle (expression importante) : Vα21, 13, 38, 19 et 17.

· groupe 2 : expression 4 à 16 inférieure à celle du groupe 1 : Vα27, 26, 16, 5, 36, 29, 10, 20, 30, 6, 25, 24. Quelques familles Vα, telles que V1, V2, V3 et V8 peuvent se retrouver dans le groupe 1 ou le groupe 2 selon les individus.

. groupe 3 : gènes toujours exprimés faiblement (plus de 5 cycles de différence, avec le gène le plus exprimé) : Vα7, 14, 22, 40, 34 et 25.

Pour évaluer à quel niveau l'expression Vα est établie, une analyse complémentaire a été réalisée sur de l'ADN de thymus. On observe dans le thymus (voir figure 8B) le même profil que dans les lymphocytes T périphériques, à l'exception de V40 qui semble plus exprimé dans le thymus.

Ces différences de taux d'expression des familles Vα ajoute un biais supplémentaire dans la génération des répertoires TCR et montre l'intérêt du procédé selon l'invention, dans lequel c'est l'analyse de l'appariement V/J en tant que tel qui est effectuée.

## RÉFÉRENCES BIBLIOGRAPHIQUES

1. FINK P.J. et al., Journal of Experimental Medicine, 1978, 148, 766-775.
2. DAVIS M.M. et al., Nature, 1988, 334, 395-402.
3. SAITO H. et al., Nature, 1984, 309, 757-762.
4. GARCIA K.C. et al., Annu Rev Immunol, 1999, 17, 369-397.
5. ALLISON T.J. et al., Nature, 2001, 411, 820-824.
6. HENNECKE J. et al., Cell, 2001, 104, 1-4.
7. HAMROUNI A. et al., J Exp Med, 2003, 197, 601-614.
8. DIETRICH P.Y. et al., J Immunol, 2003, 170, 5103-5109.
9. BASSING C.H. et al., Cell, 2002, 109, suppl:S45-55.
10. CHIEN Y.H. et al., Nature, 1984, 309, 322-326.
11. SLECKMAN B.P. et al., Immunol Rev, 1998, 165, 121-130.
12. CAPONE M. et al., Proc Natl Acad Sci USA, 1998, 95, 12522-12527.
13. VON BOEHMER H. et al., Curr Opin Immunol, 1999, 11, 135-142.
14. GELLERT M., Adv Immunol, 1997, 64, 39-64.
15. OETTINGER M.A., Science, 1990, 248, 1517-1523.
16. GELLERT M., Annu Rev Biochem, 2002, 71, 101-132.
17. McBLANE J.F. et al., Cell, 1995, 83, 387-395.
18. VAN GENT D.C. et al., Cell, 1996, 85, 107-113.
19. HESSE J.E. et al., Genes Dev, 1989, 3, 1053-1061.
20. COWELL L.G. et al., J Exp Med, 2003, 197, 207-220.
21. CABANIOLS JP. et al., J Exp Med, 2001, 194, 1385-1390.
22. SAITO T. et al., J Exp Med, 1988, 168, 1003-1020.
23. CASROUGE A. et al., J Immunol, 2000, 164, 5782-5787.
24. PASQUAL N. et al., J Exp Med, 2002, 196, 1163-1173.
25. KOOP B.F. et al., Genomics, 1994, 19, 478-493.
26. GLUSMAN G. et al., Immunity, 2001, 15, 337-349.
27. MANCINI S.J. et al., J Immunol, 2001, 167, 4485-4493.
28. GALLAGHER M. et al., J Immunol, 2001, 167, 1447-1453.
29. GAHERY-SEGARD H. et al., Immunogenetics, 1996, 44, 298-305.
30. KRANGEL M.S., Nat Immunol, 2003, 4, 624-630.
31. YANCOPOULOS G.D. et al., Cell, 1986, 44, 251-259.
32. MOSTOSLAVSKY R. et al., Nat Immunol, 2003, 4, 603-606.
33. STRAHL B.D. et al., Nature, 2000, 403, 41-45.
34. McBLANE et al., Curr Biol, 2000, 10, 483-486.
35. HSIEH C.L. et al., Embo J, 1992, 11, 315-325.
36. SPICUGLIA S. et al., Mol Cell, 2002, 10, 1479-1487.
37. HUANG C. et al., J Immunol, 2001, 166, 2597-2601.
38. WANG F. et al., Proc Natl Acad Sci USA, 1998, 95, 11834-11839.
39. DAVODEAU F. et al., Embo J, 2001, 20, 4717-4729.
40. McMURRY M.T. et al., Science, 2000, 287, 495-498.
41. MAUVIEUX L. et al., Eur J Immunol, 2001, 31, 2080-2086.
42. VILLEY I. et al., Immunity, 1996, 5, 331-342.
43. ARSTILA T.P. et al., Science, 1999, 286, 958-961.
44. BASSING C.H. et al., Nature, 2000, 405, 583-586.
45. LEE A.I. et al., PloS Biol 1:E1, 2003.
46. HODGES E et al., J Clin Pathol, 2003, 56, 1-11.
47. PERNOLLET M. et al., Clin. Exp. Immunol., 2002, 130, 518-525.
48. GULWANI-AKOLKAR B. et al., J. Immunol., 1995, 154, 3843-3851.


**Revendications**

**1.** Procédé d'évaluation quantitative d'un réarrangement génétique du locus TCRAD d'un individu, comprenant au moins :

(a) l'extraction de l'ADN génomique humain, à partir d'un prélèvement sanguin ou d'une biopsie dudit individu,
(b) l'amplification d'un segment dudit ADN génomique, de taille comprise entre quelques centaines de paires de bases et plusieurs dizaines de kb, par PCR longue permettant de détecter, en une seule réaction, plusieurs réarrangements V(D)J des gènes du locus TCRAD, en présence :

* d'un ou plusieurs couples d'amorces, dont au moins un répond aux caractéristiques suivantes :

- une des amorces, dénommée amorce V, s'hybride en amont et/ou à l'extrémité 5' d'un gène V du domaine variable de la chaîne $\alpha$ d'un récepteur des cellules T ;
- l'autre amorce, dénommée amorce J, s'hybride en aval et/ou à l'extrémité 3' d'un gène J de la chaîne $\alpha$ d'un récepteur des cellules T ;

* et d'une ADN polymérase ou d'un mélange d'ADN polymérases permettant l'amplification de segments d'ADN génomique de taille comprise entre quelques centaines de paires de bases et plusieurs dizaines de kb, de préférence de taille supérieure à 10 kb et ayant une activité de correction permettant d'améliorer sensiblement l'élongation ;
ladite amplification comprenant, outre l'étape de dénaturation initiale, des cycles de dénaturation, d'hybridation et d'élongation, dans lesquels les étapes d'élongation sont effectuées au moins pendant 10 minutes à 68˚C-72˚C ;

(c) la séparation des fragments d'ADNg amplifiés et
(d) la détection des segments réarrangés ou recombinés, par une technique choisie parmi :

(i) l'utilisation d'une base marquée lors de l'amplification puis par mesure de son incorporation directement dans le gel ;
(ii) l'utilisation d'agent marquant l'ADN lors de la migration, et détection après excitation aux UV ou autre longueur d'onde appropriée ;
(iii) l'utilisation, lors de l'amplification, d'amorces marquées par des fluorochromes ou par des moyens de révélation enzymatiques.

**2.** Procédé d'évaluation quantitative du répertoire immunitaire d'un individu, comprenant une étape d'évaluation quantitative d'un réarrangement génétique du locus TCRAD dudit individu, par un procédé selon la revendication 1.

**3.** Procédé selon la revendication 1 ou la revendication 2, **caractérisé en ce qu'**à l'étape (b) d'amplification, la sélection des amorces est effectuée :

- par analyse systématique de l'ensemble du locus TCRAD humain à l'aide d'un logiciel convenable,
- sélection des amorces dont l'extrémité 3'OH est complémentaire uniquement de la région d'intérêt,
- élimination des amorces formant des auto-dimères ou des épingles à cheveux stables, notamment par une analyse avec un logiciel convenable et
- élimination des couples d'amorces formant des hybrides entre eux.

**4.** Procédé selon la revendication 3, **caractérisé en ce que** les amorces V et J des couples d'amorces V/J sont sélectionnées dans le groupe constitué par les amorces de séquences SEQ ID NO : 1-21.

**5.** Procédé selon l'une quelconque des revendications 1 à 4, **caractérisé en ce que** l'étape (b) d'amplification met également en oeuvre des amorces supplémentaires pour amplifier au moins l'un des segments suivants : segments D, segments V et J des chaînes TCR $\beta$, $\gamma$, $\delta$ et éventuellement segments des chaînes des immunoglobulines.

**6.** Procédé selon l'une quelconque des revendications 1 à 5, **caractérisé en ce qu'**à l'étape (b) d'amplification, la réaction de PCR longue (LPCR) est effectuée après purification de l'ADN ou directement sur un lysat cellulaire.

**7.** Procédé selon l'une quelconque des revendications 1 à 6, **caractérisé en ce qu'**à l'étape (b) d'amplification, les étapes d'élongation sont incrémentées de 15-20 secondes par cycle supplémentaire d'élongation.

**8.** Procédé selon l'une quelconque des revendications 1 à 7, **caractérisé en ce que** l'étape (c) de séparation des fragments d'ADN amplifiés est effectuée par migration électrophorétique sur gel, de préférence en champ pulsé.

**9.** Procédé selon l'une quelconque des revendications 1 à 7, **caractérisé en ce que** l'étape (c) de séparation des fragments d'ADN amplifiés est effectuée par séparation en microcapillaire.

**10.** Procédé de suivi d'un traitement d'une pathologie dans laquelle le répertoire immunitaire est initialement modifié, chez un sujet concerné, lequel procédé étant **caractérisé :**

- **en ce qu**'il met en oeuvre le procédé d'évaluation du répertoire immunitaire, selon l'une quelconque des revendications 2 à 9, en début de traitement,
- en ce que ledit procédé d'évaluation est réitéré à différentes phases du traitement et
- en ce que le profil du répertoire immunitaire, obtenu à chaque fois, est comparé à celui d'un répertoire immunitaire standard, afin d'évaluer la réponse dudit sujet audit traitement.

**11.** Procédé de mesure du répertoire des récepteurs des antigènes au cours des différentes phases d'une pathologie dans laquelle le répertoire immunitaire est modifié, chez un sujet concerné, lequel procédé étant **caractérisé :**

- **en ce qu**'il met en oeuvre le procédé d'évaluation du répertoire immunitaire, selon l'une quelconque des revendications 2 à 9 à différentes phases de la pathologie et
- en ce que le profil du répertoire immunitaire obtenu à chaque fois est comparé à celui d'un répertoire immunitaire standard, afin d'évaluer l'évolution de ladite pathologie.

**12.** Procédé selon l'une quelconque des revendications 1 à 11, **caractérisé en ce que** l'étape (a) du procédé est effectuée sur un échantillon constitué de lymphocytes T.

**13.** Procédé selon la revendication 12, **caractérisé en ce que** lesdits lymphocytes T sont sélectionnés dans le groupe constitué par des cellules thymiques, des lymphocytes T du sang périphérique, des lymphocytes T d'autres organes lymphoïdes, des lymphocytes T de différents organes et des lymphocytes T, issus de tumeurs ou de sites inflammatoires.

**14.** Utilisation d'une amorce sélectionnée dans le groupe constitué par les amorces oligonucléotidiques de séquences SEQ ID NO : 1-21, pour la mise en oeuvre d'un procédé selon l'une quelconque des revendications 1 à 13.

**15.** Utilisation d'une sonde sélectionnée dans le groupe constitué par les sondes oligonucléotidiques de séquences SEQ ID NO: 22-37, pour la mise en oeuvre d'un procédé selon l'une quelconque des revendications 1 à 13.

**16.** Utilisation d'un kit d'évaluation quantitative du répertoire immunitaire d'un individu, pour la mise en oeuvre d'un procédé selon l'une quelconque des revendications 1 à 13, **caractérisée en ce que** le kit comprend, outre les tampons et réactifs usuels pour mettre en oeuvre une PCR, des amorces sélectionnées dans le groupe constitué par les amorces oligonucléotidiques de séquences SEQ ID NO : 1-21.

**17.** Utilisation selon la revendication 16, **caractérisée en ce que** le kit comprend en outre des sondes sélectionnées dans le groupe constitué par les sondes oligonucléotidiques de séquences SEQ ID NO: 22-37.

## Claims

**1.** Method for the quantitative evaluation of a genetic rearrangement of the TCRAD locus of an individual, comprising at least:

(a) extracting the human genomic DNA from a blood sample or from a biopsy of said individual,
(b) amplifying a segment of said genomic DNA having a size of from several hundreds of base pairs to several tens of kb, by long PCR permitting the detection, in a single reaction, of several V(D)J rearrangements of the genes of the TCRAD locus, in the presence of:

* one or several primer pairs, of which at least one has the following characteristics:

- one of the primers, called the V primer, hybridises upstream and/or at the 5' end of a V gene of the variable domain of the $\alpha$ chain of a T cell receptor;
- the other primer, called the J primer, hybridises downstream and/or at the 3' end of a J gene of the $\alpha$ chain of a T cell receptor;

* and a DNA polymerase or a mixture of DNA polymerases permitting amplification of segments of genomic DNA having a size of from several hundreds of base pairs to several tens of kb, preferably having a size greater than 10 kb and having a correction activity permitting a substantial improvement in the elongation; said amplification comprising, in addition to the initial denaturing step, cycles of denaturation, hybridisation and elongation, in which the elongation steps are carried out for at least 10 minutes at 68˚C-72˚C;

(c) separating the amplified gDNA fragments and
(d) detecting the rearranged or recombined segments by a technique selected from:

(i) using a labelled base during the amplification, and then measuring its incorporation directly in the gel;
(ii) using an agent that labels the DNA during the migration, and detecting after excitation with UV or another suitable wavelength;
(iii) using, during the amplification, primers labelled by fluorochromes or by enzymatic visualisation means.

**2.** Method for the quantitative evaluation of the immune repertoire of an individual, comprising a step of quantitative evaluation of a genetic rearrangement of the TCRAD locus of said individual by a method according to claim 1.

**3.** Method according to claim 1 or claim 2, **characterised in that** in the amplification step (b), selection of the primers is carried out:

- by systematic analysis of the whole human TCRAD locus with the aid of suitable software,
- selection of the primers whose 3'-OH end is complementary solely with the region of interest,
- elimination of primers that form autodimers or stable hairpins, especially by analysis using suitable software, and
- elimination of the primer pairs that form hybrids with one another.

**4.** Method according to claim 3, **characterised in that** the V and J primers of the V/J primer pairs are selected from the group constituted by the primers of sequences SEQ ID NO: 1-21.

**5.** Method according to any one of claims 1 to 4, **characterised in that** the amplification step (b) also uses additional primers to amplify at least one of the following segments: D segments, V and J segments of the β, γ, δ TCR chains, and optionally segments of the immunoglobulin chains.

**6.** Method according to any one of claims 1 to 5, **characterised in that** in the amplification step (b), the long PCR reaction (LPCR) is carried out after purification of the DNA or directly on a cell lysate.

**7.** Method according to any one of claims 1 to 6, **characterised in that** in the amplification step (b), the elongation steps are incremented by 15-20 seconds per additional elongation cycle.

**8.** Method according to any one of claims 1 to 7, **characterised in that** step (c) of separation of the amplified DNA fragments is carried out by gel electrophoretic migration, preferably pulsed-field gel electrophoretic migration.

**9.** Method according to any one of claims 1 to 7, **characterised in that** step (c) of separation of the amplified DNA fragments is carried out by microcapillary separation.

**10.** Method for monitoring a treatment of a pathology in which the immune repertoire is initially modified, in a subject in need thereof, said method being **characterised in that**:

- it uses the method for evaluation of the immune repertoire according to any one of claims 2 to 9 at the start of the treatment,
- said evaluation method is repeated at different phases of the treatment, and
- the profile of the immune repertoire obtained each time is compared with that of a standard immune repertoire in order to evaluate the response of said subject to said treatment.

**11.** Method for measuring the repertoire of the antigen receptors during the different phases of a pathology in which the immune repertoire is modified, in a subject in need thereof, said method being **characterised in that**:

- it uses the method for evaluation of the immune repertoire according to any one of claims 2 to 9 at different phases of the pathology, and
- the profile of the immune repertoire obtained each time is compared with that of a standard immune repertoire in order to evaluate the evolution of said pathology.

12. Method according to any one of claims 1 to 11, **characterised in that** step (a) of the method is carried out on a sample composed of T lymphocytes.

13. Method according to claim 12, **characterised in that** said T lymphocytes are selected from the group constituted by thymus cells, T lymphocytes of the peripheral blood, T lymphocytes of other lymphoid organs, T lymphocytes of various organs, and T lymphocytes from tumours or inflammatory sites.

14. Use of a primer selected from the group constituted by the oligonucleotide primers of sequences SEQ ID NO: 1-21, for carrying out a method according to any one of claims 1 to 13.

15. Use of a probe selected from the group constituted by the oligonucleotide probes of sequences SEQ ID NO: 22-37, for carrying out a method according to any one of claims 1 to 13.

16. Use of a kit for quantitative evaluation of the immune repertoire of an individual, for carrying out a method according to any one of claims 1 to 13, **characterised in that**, in addition to the conventional buffers and reagents for carrying out a PCR, the kit comprises primers selected from the group constituted by the oligonucleotide primers of sequences SEQ ID NO: 1-21.

17. Use according to claim 16, **characterised in that** the kit further comprises probes selected from the group constituted by the oligonucleotide probes of sequences SEQ ID NO: 22-37.

## Patentansprüche

1. Verfahren zur quantitativen Evaluierung einer genetischen Umlagerung des Locus TCRAD eines Individuums, umfassend wenigstens:

   (a) die Extraktion der humanen genomischen DNA ausgehend von einer Blutprobe oder eine Biopsie des Individuums,
   (b) die Amplifikation eines Segments der genomischen DNA mit einer Größe zwischen einigen hundert Basenpaaren und mehreren zehn kb durch long-PCR, die erlaubt, mehrere Umlagerungen V(D)J der Gene des Locus TCRAD in einer einzigen Reaktion zu detektieren, und zwar in Gegenwart:

      * eines Primerpaars oder mehrerer Primerpaare, von denen wenigstens eines den folgenden Charakteristika entspricht:

         - einer der Primer, Primer V genannt, hybridisiert stromaufwärts und/oder am 5'-Ende eines Gens V der variablen Domäne der $\alpha$-Kette eines Rezeptors der T-Zellen;
         - der andere Primer, Primer J genannt, hybridisiert stromabwärts und/oder am 3'-Ende eines Gens J der $\alpha$-Kette eines Rezeptors der T-Zellen;

      * einer DNA-Polymerase oder eines Gemisches von DNA-Polymerasen, die/das die Amplifikation von genomischen DNA-Segmenten einer Größe zwischen einigen hundert Basenpaaren und mehreren zehn kb, vorzugsweise einer Größe über 10 kb erlaubt und eine Korrekturaktivität hat, die es ermöglicht, die Verlängerung deutlich zu verbessern;
      wobei die Amplifikation außer der Stufe der Anfangsdenaturierung Zyklen der Denaturierung, Hybridisierung und Verlängerung umfasst, bei denen die Stufen der Verlängerung wenigstens während 10 Minuten bei 68°C-72°C durchgeführt werden;

   (c) die Abtrennung der amplifizierten gDNA-Fragmente und
   (d) die Detektion der umgelagerten oder rekombinierten Segmente durch eine Technik, ausgewählt aus:

      (i) die Verwendung einer markierten Base während der Amplifikation, danach durch Messung ihres Einbaus direkt in Gel;
      (ii) Verwendung eins Mittels, das die DNA markiert, während der Migration und Detektion nach Anregung mit UV oder einer geeigneten anderen Wellenlänge;

(iii) Verwendung von Primern bei der Amplifikation, die durch Fluorochrome oder durch Mittel zur enzymatischen Entwicklung markiert sind.

2. Verfahren zur quantitativen Evaluierung des Immunrepertoires eines Individuums, das eine Stufe der quantitativen Evaluierung einer genetischen Umlagerung des Locus TCRAD eines Individuums durch ein Verfahren nach Anspruch 1 umfasst.

3. Verfahren nach Anspruch 1 oder Anspruch 2, **dadurch gekennzeichnet, dass** in der Stufe (b) der Amplifikation die Auswahl der Primer durchgeführt wird:

- durch systematische Analyse der Gesamtheit des humanen Locus TCRAD mit Hilfe einer geeigneten Software,
- Auswahl von Primern, deren Ende 3'OH einzigartig komplementär zu der Region von Interesse ist,
- Eliminierung von Primern, die stabile Autodimere oder Haarnadelschleifen bilden, insbesondere durch Analyse mit einer geeigneten Software und
- Eliminierung der Primerpaare, die untereinander Hybride bilden.

4. Verfahren nach Anspruch 3, **dadurch gekennzeichnet, dass** die Primer V und J der Primerpaare V/J in der Gruppe ausgewählt werden, die von den Primern der Sequenzen SEQ ID NO:1-21 gebildet wird.

5. Verfahren nach einem der Ansprüche 1 bis 4, **dadurch gekennzeichnet, dass** die Stufe (b) der Amplifikation auch supplementäre Primer verwendet, um wenigstens eines der folgenden Segmente zu amplifizieren: Segmente D, Segmente V und J der β-, γ-, δ-Ketten des T-Zell-Rezeptors und gegebenenfalls Segmente der Ketten der Immunglobuline.

6. Verfahren nach einem der Ansprüche 1 bis 5, **dadurch gekennzeichnet, dass** in der Stufe (b) der Amplifikation die Reaktion der long-PCR (LPCR) nach Reinigung der DNA oder direkt an einem Zelllysat durchgeführt wird.

7. Verfahren nach einem der Ansprüche 1 bis 6, **dadurch gekennzeichnet, dass** in der Stufe (b) der Amplifikation die Stufen der Verlängerung 15-20 Sekunden pro zusätzlichem Verlängerungszyklus zunehmen.

8. Verfahren nach einem der Ansprüche 1 bis 7, **dadurch gekennzeichnet, dass** die Stufe (c) der Trennung der amplifizierten DNA-Fragmente durch elektrophoretische Wanderung auf Gel, vorzugsweise in einem gepulsten Feld, durchgeführt wird.

9. Verfahren nach einem der Ansprüche 1 bis 7, **dadurch gekennzeichnet, dass** die Stufe (c) der Trennung der amplifizierten DNA-Fragmente durch Trennung in einer Mikrokapillare durchgeführt wird.

10. Verfahren zur Überwachung einer Behandlung einer Pathologie, bei der das Immunrepertoire zunächst modifiziert ist, bei einem betroffenen Subjekt, wobei das Verfahren **dadurch gekennzeichnet ist:**

- **dass** es das Verfahren zur Evaluierung des Immunrepertoires nach einem der Ansprüche 2 bis 9 zu Behandlungsbeginn durchführt,
- **dass** das Verfahren zur Evaluierung zu verschiedenen Phasen der Behandlung wiederholt wird und
- **dass** das Profil des Immunrepertoires, das bei jedem Mal erhalten wird, mit dem eines Standard-Immunrepertoires verglichen wird, um die Reaktion des Subjekts auf die Behandlung zu evaluieren.

11. Verfahren zur Messung des Repertoires der Rezeptoren der Antigene im Verlauf verschiedener Phasen einer Pathologie, bei der das Immunrepertoire modifiziert ist, bei einem betroffenen Subjekt, wobei das Verfahren **dadurch gekennzeichnet ist:**

- **dass** es das Verfahren zur Evaluierung des Immunrepertoires nach einem der Ansprüche 2 bis 9 zu verschiedenen Phasen der Pathologie verwendet und
- **dass** das Profil des Immunrepertoires, das jedes Mal erhalten wird, mit demjenigen eines Standard-Immunrepertoires verglichen wird, um die Entwicklung der Pathologie zu evaluieren.

12. Verfahren nach einem der Ansprüche 1 bis 11, **dadurch gekennzeichnet, dass** die Stufe (a) des Verfahrens an einer Probe durchgeführt wird, die von T-Lymphozyten gebildet wird.

13. Verfahren nach Anspruch 12, **dadurch gekennzeichnet, dass** die T-Lymphozyten aus der Gruppe, bestehend aus Thymuszellen, T-Lymphozyten des peripheren Bluts, T-Lymphozyten anderer lymphoider Organe, T-Lymphozyten verschiedener Organe und T-Lymphozyten, die aus Tumoren oder inflammatorischen Stellen stammen, ausgewählt werden.

14. Verwendung eines Primers, ausgewählt aus der Gruppe, bestehend aus den Oligonukleotidprimern der Sequenzen SEQ ID NO:1-21, für die Durchführung eines Verfahrens nach einem der Ansprüche 1 bis 13.

15. Verwendung einer Sonde, ausgewählt aus der Gruppe, bestehend aus Oligonukleotidsonden der Sequenzen SEQ ID NO:22-37, für die Durchführung eines Verfahrens nach einem der Ansprüche 1 bis 13.

16. Verwendung eines Kits zur quantitativen Evaluierung des Immunrepertoires eines Individuums für die Durchführung eines Verfahrens nach einem der Ansprüche 1 bis 13, **dadurch gekennzeichnet, dass** das Kit außer den Puffern und üblichen Reagenzien zur Durchführung einer PCR Primer umfasst, die aus der Gruppe, bestehend aus den Oligonukleotidprimern der Sequenzen SEQ ID NO:1-21, umfasst.

17. Verwendung nach Anspruch 16, **dadurch gekennzeichnet, dass** das Kit außerdem Sonden umfasst, die aus der Gruppe, die aus Oligonukleotidsonden der Sequenzen SEQ ID NO:22-37 besteht, ausgewählt sind.

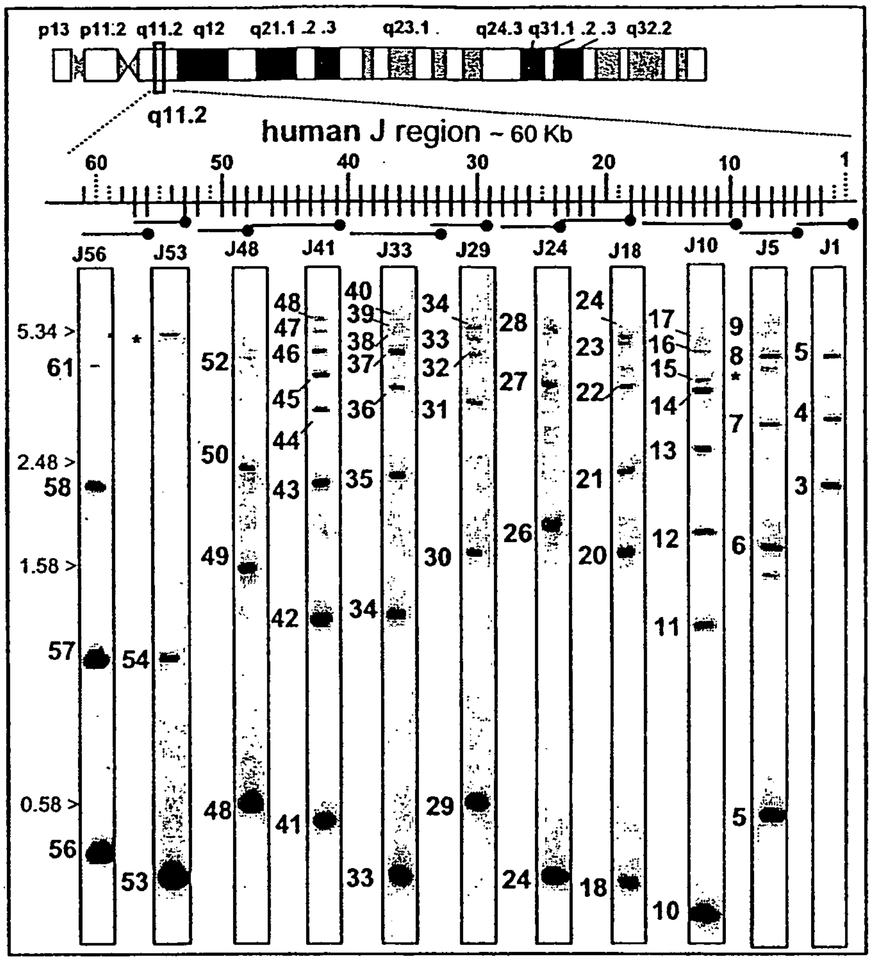
p13 p11.2 q11.2 q12 q21.1 .2 .3 q23.1 q24.3 q31.1 .2 .3 q32.2
q11.2
human J region ~ 60 Kb
60
50
40
30
20
10
1
J56
J53
J48
J41
J33
J29
J24
J18
J10
J5
J1
5.34 >
2.48 >
1.58 >
0.58 >

FIGURE 1

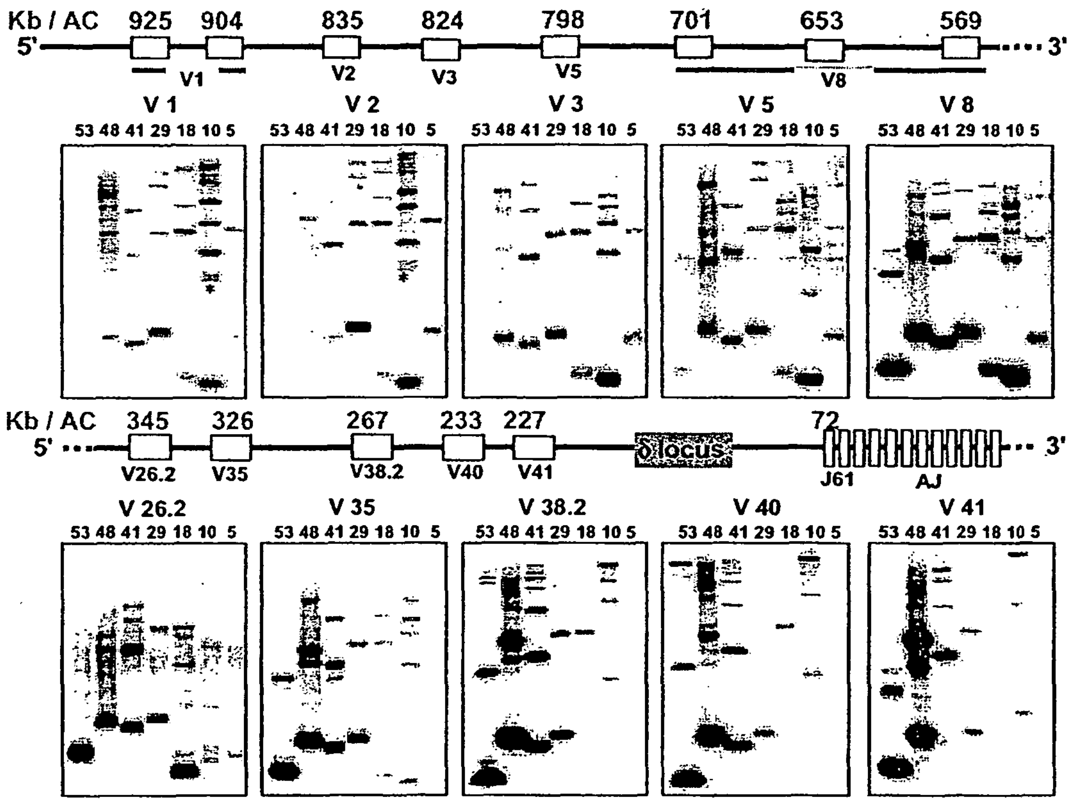
Kb / AC
925
904
835
824
798
701
653
569
5'
3'
V1
V2
V3
V5
V8
V 1
V 2
V 3
V 5
V 8
53 48 41 29 18 10 5
Kb / AC
345
326
267
233
227
72
5'
3'
V26.2
V35
V38.2
V40
V41
δ locus
J61
AJ
V 26.2
V 35
V 38.2
V 40
V 41

FIGURE 2

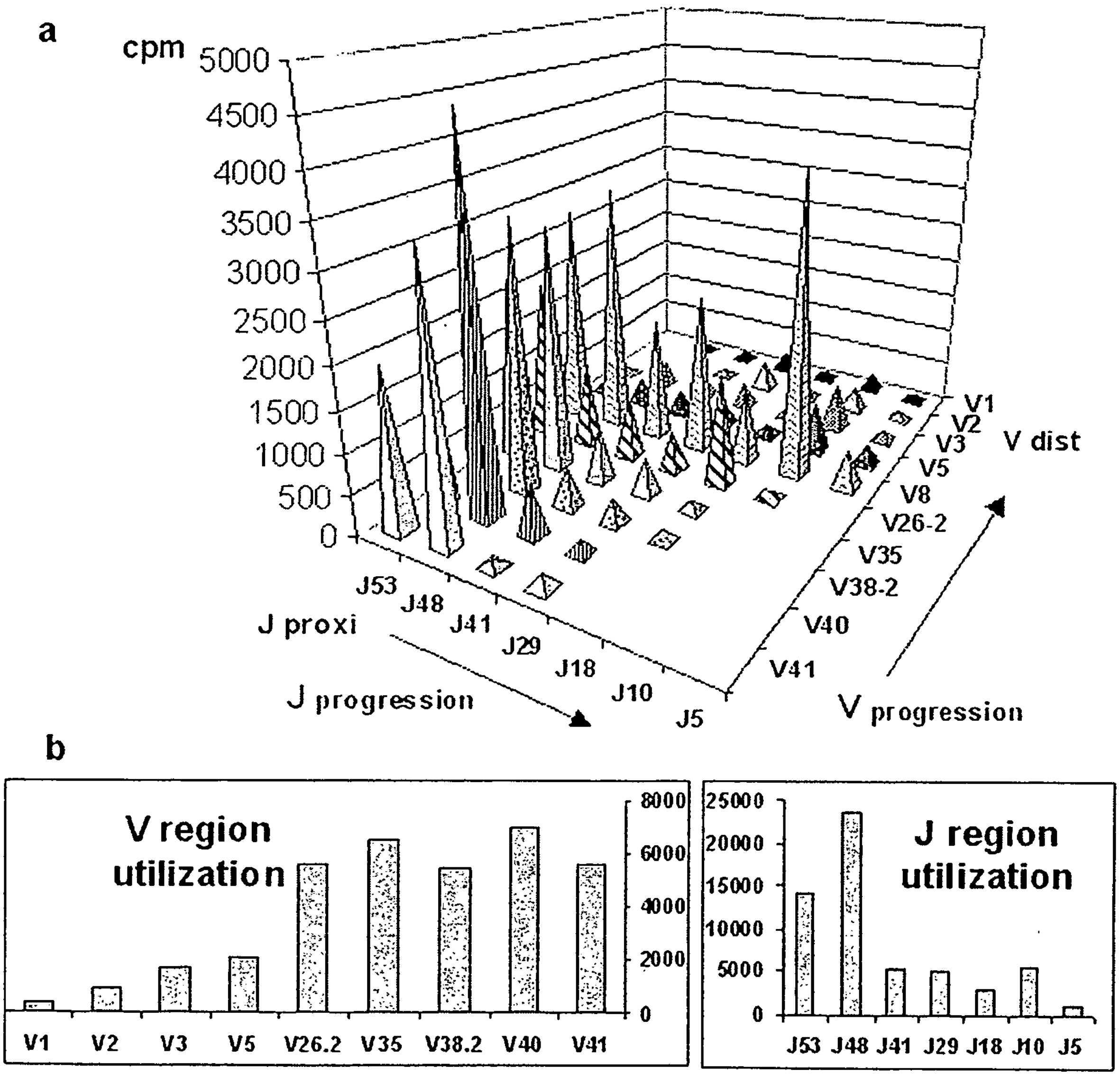
a
cpm
5000
4500
4000
3500
3000
2500
2000
1500
1000
500
0
J53
J48
J41
J29
J18
J10
J5
J proxi
J progression
V1
V2
V3
V5
V8
V26-2
V35
V38-2
V40
V41
V dist
V progression
b
V region utilization
8000
6000
4000
2000
0
V1
V2
V3
V5
V26.2
V35
V38.2
V40
V41
J region utilization
25000
20000
15000
10000
5000
0
J53
J48
J41
J29
J18
J10
J5

FIGURE 3

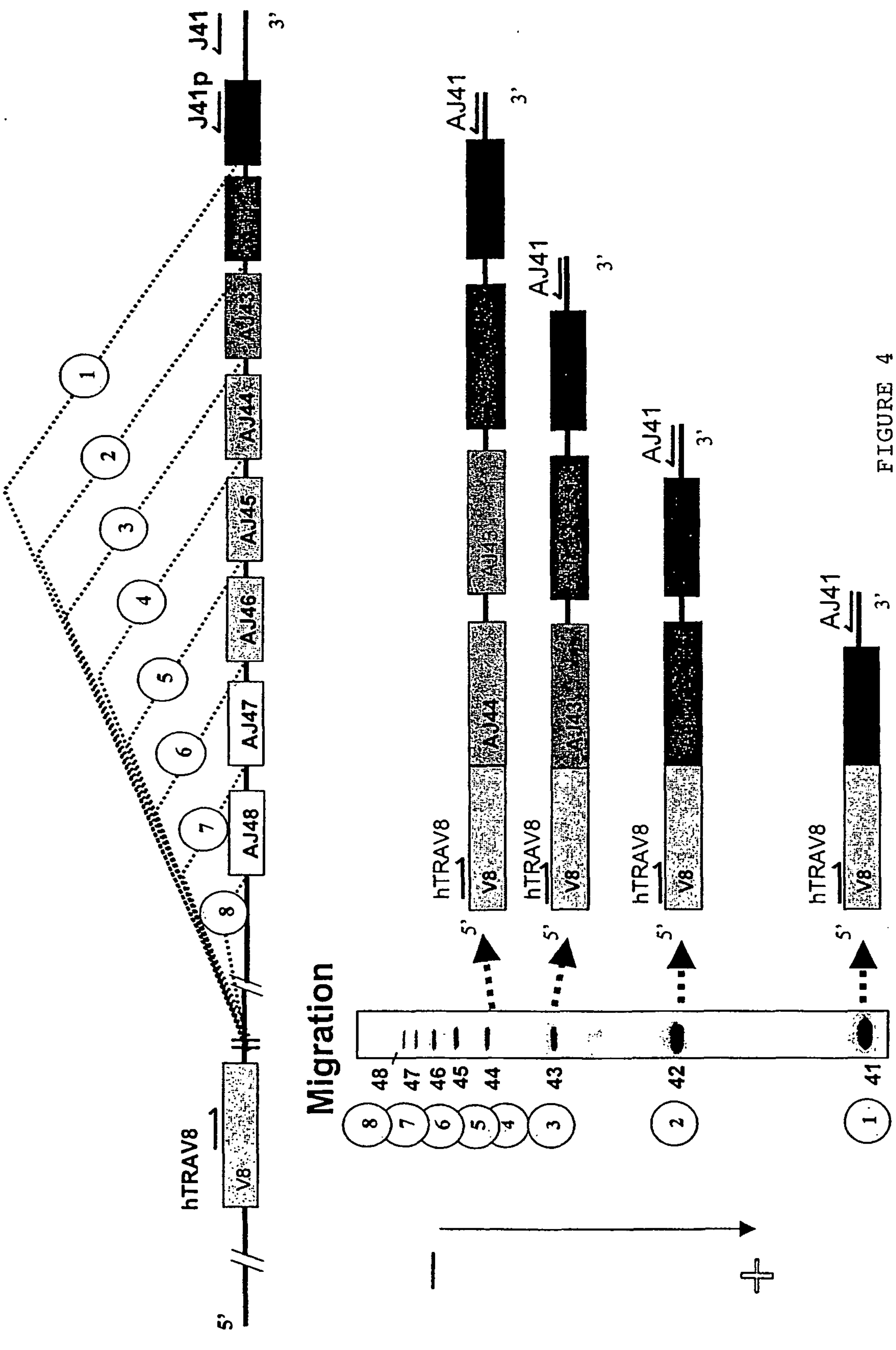
5'
hTRAV8
V8
AJ48
AJ47
AJ46
AJ45
AJ44
AJ43
J41p
J41
3'
1
2
3
4
5
6
7
8
Migration
48
47
46
45
44
43
42
41
AJ41
-
+

FIGURE 4

| hV gene | Heptamer | % | Spacer | % | Nonamer | % | RSS Score | Dist/Co (Kb) | Signal Qlity | J tropism |
|---|---|---|---|---|---|---|---|---|---|---|
| hV1-1 | CACAGTG | 100 | ACTATGAGGCCTCCTTAACTGTG | 56 | CCAAAAATTC | 56 | 72 | 925 | 1604 | J10 |
| hV1-2 | CACGGTG | 86 | ACTATGAGGCCTCTTTAGCTGCA | 63 | CCAAAAATTC | 56 | 69 | 904 | | J10 |
| hV2 | CACAGAG | 86 | GCAGGGAACCCATGAAGAGCTGA | 56 | ACAGAAACA | 78 | 75 | 835 | 1637 | J10 |
| hV3 | CACACTG | 86 | ATAGGGGCTGCAGGGGGAGCAGA | 56 | ACACAAACT | 89 | 80 | 824 | 1940 | J10 |
| hV5 | CACATTG | 86 | CTTCTCAGGCACCTGTATCCTGT | 94 | ACCCAAACC | 100 | 93 | 798 | 2406 | J10 |
| hV8-2 | CACAGTG | 100 | CTTGAGACTGCAGGAGAGCTGAA | 50 | CACAAGCCT | 33 | 63 | 701 | | |
| hV8-4 | CACAGTG | 100 | CTTGAGACTGCAGGAGAGCTGAA | 50 | CATAAACCT | 33 | 63 | 653 | 15830 | All J area |
| hV8-6 | CACAGTG | 100 | CCTGAGACTGCAGGAGAGCTGAA | 44 | CACAAACCT | 44 | 65 | 569 | | |
| hV26-2 | CACAGTG | 100 | GGACAGATGGGGCTGCAGCTGTG | 56 | CAATATCTC | 33 | 64 | 345 | 5638 | J48 |
| hV35 | CACAGTG | 100 | CTCCCCAAACACCTGCAGCCTGT | 94 | ACTCAAACT | 78 | 90 | 326 | 6520 | J48 |
| hV38-2 | CACAGTG | 100 | AGACAAGCAACAGGGAGAGGCTT | 31 | ACAGAAACC | 89 | 78 | 267 | 11008 | J48 |
| hV40 | CACTGTG | 86 | TTAAAAGCACAGTGGGAGCTATA | 44 | CAAAAAACCT | 44 | 60 | 233 | 6930 | J48 |
| hV41 | CACAGTG | 100 | CTCCCCAGGCACCTGGAGCCCGT | 94 | ACCTAAACT | 78 | 90 | 227 | 5630 | J48 |
| Consensus | CACAGTG | | -T---CAG-CA-CTG-AACCTGT<br>G GC GG | | ACACAAACC<br>C | | % | | | |

FIGURE 5

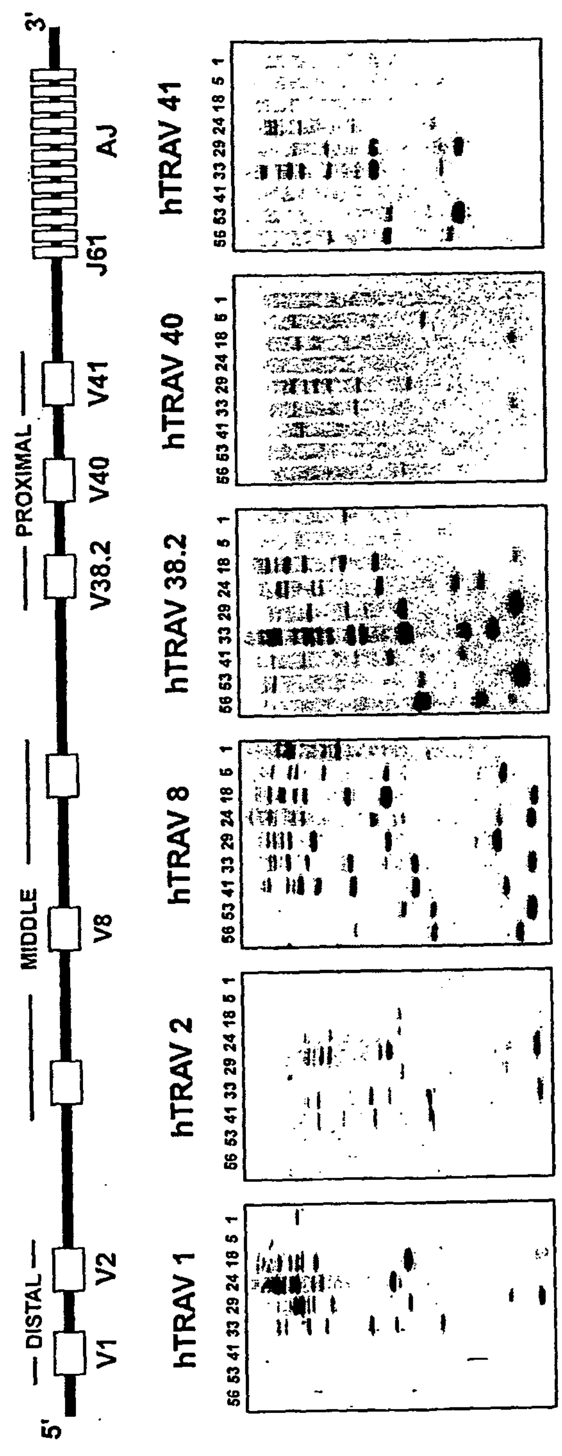
5'
DISTAL
V1
V2
MIDDLE
V8
PROXIMAL
V38.2
V40
V41
J61
AJ
3'
hTRAV 1
hTRAV 2
hTRAV 8
hTRAV 38.2
hTRAV 40
hTRAV 41
56 53 41 33 29 24 18 5 1

FIGURE 6

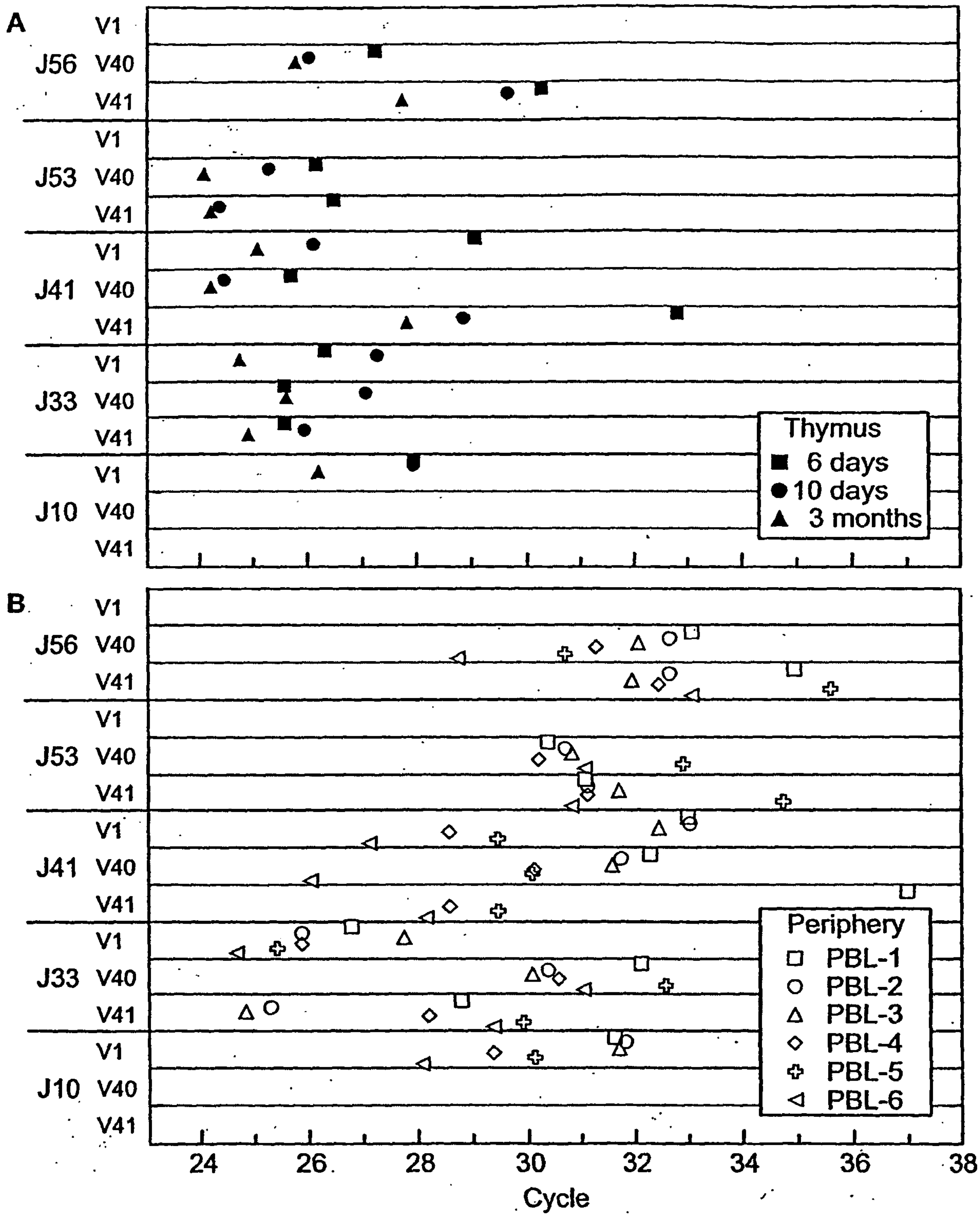
A
J56
J53
J41
J33
J10
V1
V40
V41
Thymus
6 days
10 days
3 months
B
Periphery
PBL-1
PBL-2
PBL-3
PBL-4
PBL-5
PBL-6
24
26
28
30
32
34
36
38
Cycle

FIGURE 7

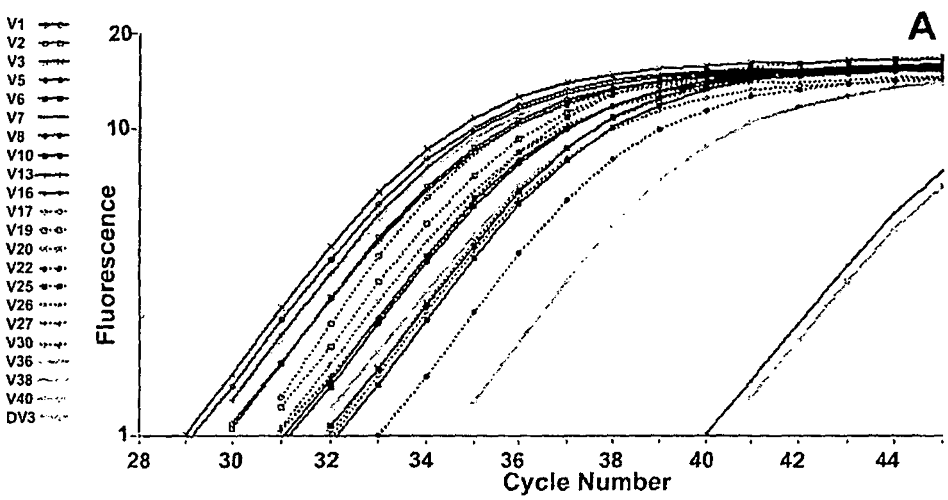


B

| | | | | | | | | | | | | | |
|---|---|---|---|---|---|---|---|---|---|---|---|---|---|
| **PBL5** | 9<br>19<br>38 | 2<br>3<br>13<br>21 | 1<br>17 | 6<br>8<br>20<br>26<br>27 | 16<br>24<br>30<br>36 | 10<br>25<br>29 | 40 | | 7<br>14 | 22 | 34 | | |
| **PBL 3** | 19<br>21<br>38 | 1<br>9<br>13<br>17 | 3 | 5<br>8<br>25 | 2<br>6<br>16<br>20<br>26<br>27<br>30 | 10<br>24 | 29 | 34<br>40 | 14<br>22 | | | | |
| **PBL 2** | 13 | 38 | 17<br>19<br>21 | 3<br>24<br>27 | 16<br>20<br>25<br>36 | 1<br>8<br>20<br>25 | 2<br>5<br>26<br>29 | 6<br>10<br>22<br>34 | | | 14<br>40 | | |
| **PBL 1** | 3<br>8<br>13<br>21<br>38 | 1<br>2<br>17<br>19 | 5<br>16<br>26<br>27<br>36 | 6<br>10<br>20<br>24<br>25<br>29<br>30 | 14<br>22 | 40 | | | | 35 | | | 7 |
| **Thymus** 3 months | 21<br>38 | 1<br>2<br>13<br>17 | 3<br>9<br>19<br>26<br>36 | 6<br>8<br>10<br>16<br>25<br>27 | 5<br>20<br>22<br>40 | 24<br>29<br>30 | 14<br>34 | | | | | | 7<br>35 |
| **Relative cycle** | | +1 | +2 | +3 | +4 | +5 | +6 | +7 | +8 | +9 | +10 | +11 | +12 |

FIGURE 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2671356
- US 5635354 A
- WO 02084567 A
- WO 2004033728 A
- US 6410277 B
- FR 0314289


### Littérature non-brevet citée dans la description


- **Panzara M.A. et al.** *Biotechniques,* 1992, vol. 12 (5), 728-735
- **Langerak A.W. et al.** *Blood,* 2001, vol. 98 (1), 165-173
- **Pannetier et al.** *Immunol. Today,* 1995, vol. 16 (4), 176-181
- **Sambrook J. ; Fritsch EF. ; Maniatis T.** Molecular cloning. A laboratory manual. Cold Spring Harbor, 1989
- **FINK P.J. et al.** *Journal of Experimental Medicine,* 1978, vol. 148, 766-775
- **DAVIS M.M. et al.** *Nature,* 1988, vol. 334, 395-402
- **SAITO H. et al.** *Nature,* 1984, vol. 309, 757-762
- **GARCIA K.C. et al.** *Annu Rev Immunol,* 1999, vol. 17, 369-397
- **ALLISON T.J. et al.** *Nature,* 2001, vol. 411, 820-824
- **HENNECKE J. et al.** *Cell,* 2001, vol. 104, 1-4
- **HAMROUNI A. et al.** *J Exp Med,* 2003, vol. 197, 601-614
- **DIETRICH P.Y. et al.** *J Immunol,* 2003, vol. 170, 5103-5109
- **BASSING C.H. et al.** *Cell,* 2002, vol. 109, S45-55
- **CHIEN Y.H. et al.** *Nature,* 1984, vol. 309, 322-326
- **SLECKMAN B.P. et al.** *Immunol Rev,* 1998, vol. 165, 121-130
- **CAPONE M. et al.** *Proc Natl Acad Sci USA,* 1998, vol. 95, 12522-12527
- **VON BOEHMER H. et al.** *Curr Opin Immunol,* 1999, vol. 11, 135-142
- **GELLERT M.** *Adv Immunol,* 1997, vol. 64, 39-64
- **OETTINGER M.A.** *Science,* 1990, vol. 248, 1517-1523
- **GELLERT M.** *Annu Rev Biochem,* 2002, vol. 71, 101-132
- **McBLANE J.F. et al.** *Cell,* 1995, vol. 83, 387-395
- **VAN GENT D.C. et al.** *Cell,* 1996, vol. 85, 107-113
- **HESSE J.E. et al.** *Genes Dev,* 1989, vol. 3, 1053-1061
- **COWELL L.G. et al.** *J Exp Med,* 2003, vol. 197, 207-220
- **CABANIOLS JP. et al.** *J Exp Med,* 2001, vol. 194, 1385-1390
- **SAITO T. et al.** *J Exp Med,* 1988, vol. 168, 1003-1020
- **CASROUGE A. et al.** *J Immunol,* 2000, vol. 164, 5782-5787
- **PASQUAL N. et al.** *J Exp Med,* 2002, vol. 196, 1163-1173
- **KOOP B.F. et al.** *Genomics,* 1994, vol. 19, 478-493
- **GLUSMAN G. et al.** *Immunity,* 2001, vol. 15, 337-349
- **MANCINI S.J. et al.** *J Immunol,* 2001, vol. 167, 4485-4493
- **GALLAGHER M. et al.** *J Immunol,* 2001, vol. 167, 1447-1453
- **GAHERY-SEGARD H. et al.** *Immunogenetics,* 1996, vol. 44, 298-305
- **KRANGEL M.S.** *Nat Immunol,* 2003, vol. 4, 624-630
- **YANCOPOULOS G.D. et al.** *Cell,* 1986, vol. 44, 251-259
- **MOSTOSLAVSKY R. et al.** *Nat Immunol,* 2003, vol. 4, 603-606
- **STRAHL B.D. et al.** *Nature,* 2000, vol. 403, 41-45
- **McBLANE et al.** *Curr Biol,* 2000, vol. 10, 483-486
- **HSIEH C.L. et al.** *Embo J,* 1992, vol. 11, 315-325
- **SPICUGLIA S. et al.** *Mol Cell,* 2002, vol. 10, 1479-1487
- **HUANG C. et al.** *J Immunol,* 2001, vol. 166, 2597-2601
- **WANG F. et al.** *Proc Natl Acad Sci USA,* 1998, vol. 95, 11834-11839

- **DAVODEAU F. et al.** *Embo J,* 2001, vol. 20, 4717-4729
- **McMURRY M.T. et al.** *Science,* 2000, vol. 287, 495-498
- **MAUVIEUX L. et al.** *Eur J Immunol,* 2001, vol. 31, 2080-2086
- **VILLEY I. et al.** *Immunity,* 1996, vol. 5, 331-342
- **ARSTILA T.P. et al.** *Science,* 1999, vol. 286, 958-961
- **BASSING C.H. et al.** *Nature,* 2000, vol. 405, 583-586
- **LEE A.I. et al.** *PloS Biol,* 2003, vol. 1, E1
- **HODGES E et al.** *J Clin Pathol,* 2003, vol. 56, 1-11
- **PERNOLLET M et al.** *Clin. Exp. Immunol.,* 2002, vol. 130, 518-525
- **GULWANI-AKOLKAR B. et al.** *J. Immunol.,* 1995, vol. 154, 3843-3851